# EUROPEAN PATENT APPLICATION

(11) **EP 4 035 682 A1**
(43) Date of publication of application: **03.08.2022**
(21) Application number: 21305116.2
(22) Date of filing: 29.01.2021
(51) Int. Cl.: A61K 41/00, A61K 47/64, A61P 17/00, A61P 27/02

(54) **BIOCONJUGATE COMPRISING A PHOTOSENSITIZER UNIT, METHOD FOR ITS PREPARATION AND USE THEREOF**

(71) Applicant: UNIVERSITE DE BORDEAUX, 33000 Bordeaux (FR); Institut Polytechnique de Bordeaux, 33402 Talence Cedex (FR); Centre national de la recherche scientifique, 75016 Paris (FR); Universidad Autónoma De Madrid, 28049 Madrid (ES)
(72) Inventor: LECOMMANDOUX, Sébastien, 33610 CANEJAN (FR); GARANGER, Elisabeth, 33400 TALENCE (FR); IBRAHIMOVA, Vusala, 33400 TALENCE (FR); TORRES-CEBADA, Tomas, 28007 MADRID (ES); GONZALEZ-DELGADO, José Antonio, 21004 HUELVA (ES)
(74) Representative: Ipside

(57) **Abstract**

The invention relates to a bioconjugate comprising a photosensitizer unit, in particular a phthalocyanine unit, and an elastin-like polypeptide conjugated thereto, said elastin-like polypeptide containing at least one occurrence of a monomeric unit having the formula Xaa₁PXaa₂Xaa₃G, wherein P represents a prolyl residue, G represents a glycyl residue, Xaa₂ represents a glycyl residue or an alanyl residue and either Xaa₁ represents a valyl residue and Xaa₃ represents a sulfur-containing amino acid residue having a side chain of formula -(CH₂)_{z}-S-R' or Xaa₃ represents a valyl residue and Xaa₁ represents a sulfur-containing amino acid residue having a side chain of formula -(CH₂)_{z}-S-R'. This bioconjugate proves particularly useful for the treatment of diseases by photodynamic therapy.

## Description

### FIELD OF THE INVENTION

The invention lies in the field of therapy, in particular of photodynamic therapy.

More particularly, the invention relates to a bioconjugate comprising a photosensitizer and a biomacromolecular carrier, that proves to be particularly useful as a photosensitizing component for photodynamic therapy, in particular for the treatment of skin diseases, ocular diseases, or any disease for the treatment of which photodynamic therapy can be applied. The invention also relates to a method of preparing such a bioconjugate, as well as to a use thereof for treating a disease by photodynamic therapy treatment. Another object of the invention is a pharmaceutical composition comprising such a bioconjugate in a pharmaceutically acceptable vehicle.

### BACKGROUND OF THE INVENTION

Photodynamic therapy (PDT) is an attractive non-invasive disease treatment alternative with high selectivity. Among others, photodynamic therapy can be used for the treatment of cancer since it shows lower side effects as compared to conventional methods like surgery, chemotherapy, and radiotherapy.

Photodynamic therapy requires three interacting components: a light-activatable compound, called photosensitizer, light of appropriate wavelength and oxygen. These components, while being individually non-toxic, generate highly toxic reactive oxygen species (ROS), such as singlet oxygen (¹O₂), when used in combination: as a result of the selective activation of the photosensitizer, accumulated at a tumor site, by light at an appropriate wavelength, typically of between 600 to 800 nm, energy or electron is transferred from the excited triplet state of the activated photosensitizer to nearby molecular oxygen. The generated reactive oxygen species cause multiple oxidative damages to the surrounding microenvironment. This has a double effect in the particular case of cancer therapy. Impairing malignant cells' metabolic function leads to apoptosis and/or necrosis of these cells. Small scale inflammations, occurring after photodynamic therapy anticancer treatment, have also been shown to activate the natural immune system against malignant cells, resulting in the complete destruction of the tumor and limiting the risk of tumor recovery.

Together with low dark toxicity, the success of photodynamic therapy mainly relies on the photophysical properties of the photosensitizer used, and in particular of its extinction coefficient (ε), singlet oxygen (¹O₂) generation quantum yield (Φ_{Δ}), etc., and on its effective accumulation at the target site.

Among the photosensitizers proposed by the prior art, phthalocyanines, a class of second-generation photosensitizers, are considered as very potent. Phthalocyanines are synthetic derivatives of porphyrins, composed of four pyrrole subunits each one fused with an additional benzo ring and linked *via* nitrogen atoms. They present several photophysical properties that make them ideal compounds for photodynamic therapy. In particular, they are robust and very versatile molecules with a strong absorption at 670 - 770 nm, which yields high singlet oxygen production. Several phthalocyanine-based photosensitizers, such as Photosens, Photocyanine, Pc 4, IRDye^{®}700DX, have been approved for clinical use or are in advanced stages of clinical trials.

Further development of these phototherapeutic agents, both at the molecular level and as nanometer-sized formulations, is currently of strong interest.

Due to their intrinsic hydrophobicity, the phthalocyanine molecules have a strong tendency to aggregate in physiological medium. To allow the systemic administration of this type of photosensitizers, it has been proposed by the prior art to develop derivatives of phthalocyanines with hydrophilic properties. Nevertheless, uncontrolled aggregation tendency of phthalocyanine backbones, as well as their low molar mass, leads to uncontrolled biodistribution of this type of photosensitizers, and to low accumulation at the tumor site in the particular case of application of photodynamic therapy to the treatment of cancer.

Several studies, in particular as described in van der Meel et al., Nature Nanotechnology 2019, 14(11), 1007-1017 or in Ibrahimova et al., Nanoscale 2017, 9(31), 11180-11186, have else shown that macromolecular conjugates or nanocarriers of photosensitizers improve their biodistribution, bioavailability and therapeutic outcome by increasing their solubility in physiological fluids and plasma half-life and by avoiding their fast renal clearance. However, the efficiency of the photosensitizer-delivery systems proposed up to date is still not satisfactory enough, in particular when the photosensitizer is a phthalocyanine.

The present invention aims to overcome the drawbacks of the systems for the delivery of phthalocyanines, and more generally of photosensitizers, proposed by the prior art, in particular the drawbacks described above, by proposing a new technology for efficiently delivering photosensitizers at a target site, in such a way as to obtain a strong improvement of the clinical effectiveness of photodynamic therapy treatments, in particular of those based on phthalocyanines.

It has been discovered by the inventors that this objective, and much more, can be achieved by covalently coupling a photosensitizer with a particular polypeptide, more precisely with a recombinant elastin-like polypeptide presenting at least one specific sulfur-containing amino acid residue (more precisely, an amino acid residue having a side chain containing a thioether group) and showing lower critical solution temperature (LCST) phase transition behavior. The resulting hydrophobic bioconjugate with low LCST spontaneously self-assembles into particles at physiological body temperature. It is however advantageously turned into a hydrophilic compound, soluble at physiological body temperature, upon oxidation by the very same reactive oxygen species that are generated by the photosensitizer when the latter is activated during the photo-irradiation step of the photodynamic therapy process. Then, specific oxidation into sulfoxide of the thioether group of the sulfur-containing amino acid residues results in an increase of hydrophilicity of the bioconjugate that displays a LCST to a value far above the physiological body temperature, therefore triggering the disassembly of the particles at the physiological body temperature. The bioconjugate thus obtained, in its free dissociated form, is then able to diffuse more easily into dense tissues, thereby advantageously allowing an efficient second photo-irradiation step to be carried out. Such a reactivation of the photosensitizer may then cause considerable damage deeper in the diseased tissue and maximize the therapeutic benefit of the treatment.

### SUMMARY OF THE INVENTION

According to a first aspect, the invention relates to a photosensitizer unit and an elastin-like polypeptide conjugated thereto, said elastin-like polypeptide containing at least one occurrence of a monomeric unit having the formula (A):
Xaa₁PXaa₂Xaa₃G (A)
wherein P represents a prolyl residue, G represents a glycyl residue, Xaa₂ represents a glycyl residue or an alanyl residue and:
- either Xaa₁ represents a valyl residue and Xaa₃ represents a sulfur-containing amino acid residue having a side chain of formula -(CH₂)_{z}-S-R', wherein z is equal to 1 or 2 and R' represents a linear, branched and/or cyclic C1-C22 hydrocarbon radical, saturated and/or unsaturated, aromatic or not, optionally substituted, optionally interrupted by one or more heteroatoms and/or by one or more groups comprising at least one heteroatom or group comprising at least one heteroatom,
- or Xaa₃ represents a valyl residue and Xaa₁ represents a sulfur-containing amino acid residue having a side chain of formula -(CH₂)_{z}-S-R', wherein z is equal to 1 or 2 and R' is as defined above.

In particular embodiments of the invention, the photosensitizer is selected in the group consisting of porphyrins, phthalocyanines, bodipy-based photosensitizers and methylene blue.

In particular embodiments of the invention, the photosensitizer unit is covalently attached to the elastin-like polypeptide by a linker.

The elastin-like polypeptide may have the formula (B):
Z-[VPXaa₂Xaa₃G]ₓ-OH (B)
wherein x is an integer between 1 and 200, Z represents a peptide fragment comprising 1 to 20 amino acid residues, V represents a valyl residue, P represents a prolyl residue, G represents a glycyl residue, Xaa₂ represents a glycyl residue or an alanyl residue and Xaa₃ represents a variable amino acid residue selected from the group consisting of a valyl residue and a sulfur-containing amino acid residue having a side-chain of formula -(CH₂)_{z}-S-R', z and R' being as defined above, Xaa₃ being such that the molar ratio valyl residue/sulfur-containing amino acid residue in the -[VPXaa₂Xaa₃G]ₓ peptide fragment is between 0/1 and 10/1.

Another aspect of the invention concerns a method of preparing a bioconjugate according to the invention, which comprises:
- modifying the N-terminal end of an elastin-like polypeptide containing at least one occurrence of a monomeric unit having the formula (A): Xaa₁PXaa₂Xaa₃G (A), wherein P, G, Xaa₁, Xaa₂ and Xaa₃ are as defined above, to introduce a terminal alkyne group,
- functionalizing a photosensitizer molecule to introduce an azide group,
- and realizing a Huisgen's copper(I)-catalyzed alkyne-azide cycloaddition reaction between the elastin-like polypeptide so modified and the photosensitizer so functionalized.

An alternative method of preparing a bioconjugate according to the invention comprises functionalizing a photosensitizer molecule to introduce a N-hydroxysuccinimide-activated ester group, and realizing the direct coupling of said activated group to the N-terminal amine group of an elastin-like polypeptide containing at least one occurrence of a monomeric unit having the formula (A): Xaa₁PXaa₂Xaa₃G (A), wherein P, G, Xaa₁, Xaa₂ and Xaa₃ are as defined above. Another aspect of the invention is the use of a bioconjugate according to the invention for the treatment of a disease by photodynamic therapy, in particular for the treatment of a skin disease, of an ocular disease, or of any disease for the treatment of which photodynamic therapy may prove useful. The bioconjugate of the invention may in particular be used for the treatment of cancer.

A fourth aspect of the invention is a pharmaceutical composition comprising a bioconjugate according to the invention in a pharmaceutically acceptable vehicle.

### BRIEF DESCRIPTION OF THE DRAWINGS

Figure 1 illustrates schematically different steps of a method of treating cancer by photodynamic therapy implementing a bioconjugate according to the invention.

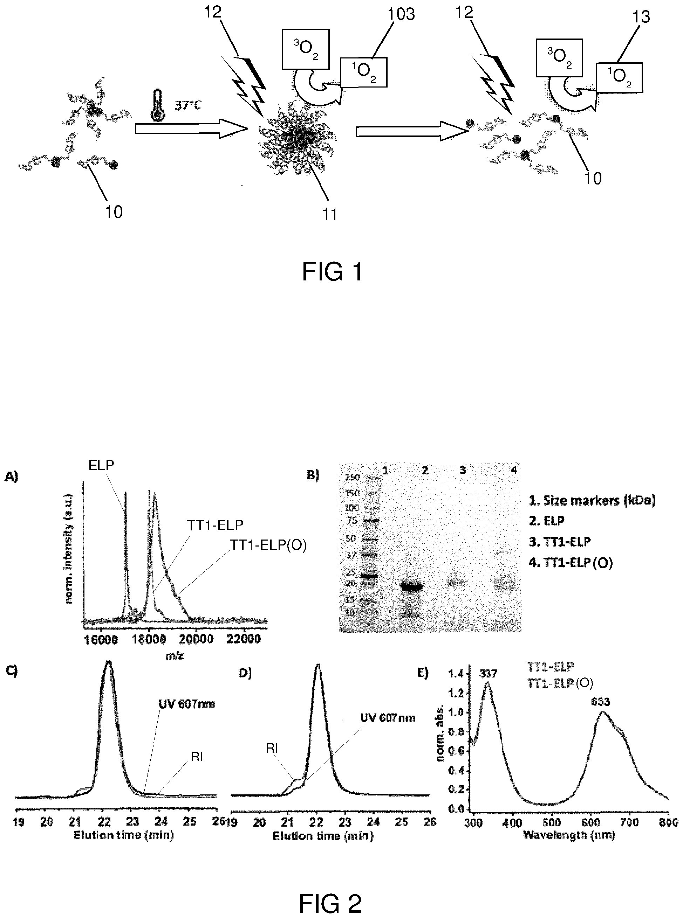

Figure 2 shows the results of characterization analyses of a bioconjugate according to the invention (TT1-ELP) and of a derivative thereof after chemical oxidation of the sulfur atoms of the methionyl residues (control compound TT1-ELP(O)). A) Superimposed MALDI mass spectra of TT1-ELP and TT1-ELP(O). The spectrum of ELP is provided for comparison. B) SDS-PAGE analysis. C) Size exclusion chromatogram of TT1-ELP in DMSO (Refractive index (RI) detection and UV detection at 607 nm). D) Size exclusion chromatogram of TT1-ELP(O) in DMSO (RI detection and UV detection at 607 nm). E) Overlapped absorption spectra of TT1-ELP and TT1-ELP(O) in PBS buffer at 25 °C.
Figure 3 shows the results of characterization analyses of a bioconjugate according to the invention (TT1-ELP) after photo-irradiation with red light. A) Stacked ¹H NMR spectra of TT1-ELP after photo-irradiation during 5, 10, 15, 20, and 30 minutes (D₂O, room temperature, 200 MHz). B) SEC chromatograms (RI detection) in DMSO of TT1-ELP before and after photoirradiation (TT1-ELP^{PhOx}). C) SDS-PAGE analysis of the bioconjugate before (TT1-ELP) and after (TT1-ELP^{PhOx}) photo-irradiation (Coomassie blue stain).
Figure 4 shows graphs representing, as measured by dynamic light scattering, the size distribution (intensity) of a bioconjugate according to the invention TT1-ELP. A) Before photo-irradiation, at 20 °C and at 37 °C. B) After photo-irradiation, at 37 °C.
Figure 5 shows, for a bioconjugate (TT1-ELP) according to the invention, results of A) dynamic light scattering taken by Cordouan (165° 657 nm, ambient temperature); B) Temperature-Controlled High-Speed AFM (liquid) images on HOPG substrate at 15 °C in PBS.
Figure 6 shows the results of characterization analyses of bioconjugates according to the invention (TT1-ELP[M₁V₃-60] and TT1-ELP[M₁V₃-80]). A) ¹H NMR spectra in D₂O at 20 °C. B) SDS-PAGE analysis.

### DETAILED DESCRIPTION OF THE INVENTION

A bioconjugate according to the invention comprises, conjugated to one another, i.e. attached to one another by a stable covalent link, either directly or via a linker:
- a photosensitizer unit
- and an elastin-like polypeptide containing at least one, preferably several, in particular between 1 and 200, occurrence(s) of a monomeric unit having the formula (A):
   Xaa₁PXaa₂Xaa₃G (A)
   wherein P represents a prolyl residue, G represents a glycyl residue, Xaa₂ represents a glycyl residue or an alanyl residue and:
   - either Xaa₁ represents a valyl residue and Xaa₃ represents a sulfur-containing amino acid residue, which may be a natural or a non-natural amino acid residue, optionally modified, having a side chain of formula -(CH₂)_{z}-S-R', wherein z is equal to 1 or 2 and R' represents a linear, branched and/or cyclic C1-C22 hydrocarbon radical, saturated and/or unsaturated, aromatic or not, optionally substituted, optionally interrupted by one or more heteroatoms and/or by one or more groups comprising at least one heteroatom or group comprising at least one heteroatom,
   - or Xaa₃ represents a valyl residue and Xaa₁ represents a sulfur-containing amino acid residue, which may be a natural or a non-natural amino acid residue, optionally modified, having a side chain of formula -(CH₂)_{z}-S-R', wherein z is equal to 1 or 2 and R' represents a linear, branched and/or cyclic C1-C22 hydrocarbon radical, saturated and/or unsaturated, aromatic or not, optionally substituted, optionally interrupted by one or more heteroatoms and/or by one or more groups comprising at least one heteroatom or group comprising at least one heteroatom.

Recombinant elastin-like polypeptides are artificial protein polymers derived from the hydrophobic domain of tropoelastin, the soluble precursor of elastin. They contain repetitive pentapeptide fragments having the amino acid sequence -Xaa₄-Pro-Xaa₅-Xaa₆-Gly- (SEQ ID No: 1), wherein Xaa₅ denotes a glycyl residue or an alanyl residue and Xaa₄ and Xaa₆ each denote a variable amino acid residue that may be any natural or non-natural amino acid residue except a prolyl residue or a derivative thereof, at least one of Xaa₄ and Xaa₆ being a valyl residue.

These nature-inspired genetically engineered recombinant elastin-like polypeptides are remarkable smart polypeptides for biomedical applications, in particular for drug delivery applications, owing to their controlled molecular weight, biocompatibility, biodegradability, non-immunogenicity and stimuli-responsive properties. In addition, they can be produced in a cost-effective way in large scale with precise control on their molecular composition.

The elastin-like polypeptide of the invention comprises at least one sulfur-containing amino acid residue having a side chain of formula -(CH₂)_{z}-S-R'. It preferably contains several of such residues, preferably regularly distributed along the polypeptide backbone, in particular between 2 and 200 of such residues.

In the present description, by convention, the phrase "sulfur-containing amino acid residue" will be used to designate a sulfur-containing amino acid residue having a side chain of formula -(CH₂)_{z}-S-R', wherein z and R' are as herein defined.

The term "photosensitizer" is herein used to designate, in a conventional way, a compound that has the capacity of transferring energy acquired upon electron excitation, promoted by light irradiation, to surrounding oxygen molecules resulting in singlet oxygen generation and other reactive oxygen species (ROS). Preferably, the photosensitizer is selected in the group consisting of phthalocyanines, porphyrins, bodipy-based photosensitizers and methylene blue.

In preferred embodiments of the invention, in the side chain of Xaa₁ or in the side chain of Xaa₃, R' represents a linear or branched alkyl group, in particular a C1-C3 alkyl group, for example a methyl group.

In the formula (A), Xaa₁ and Xaa₃ may be such that:
- Xaa₁ represents a valyl residue and Xaa₃ represents an amino acid residue selected in the group consisting of a methionyl residue, a S-alkyl-homocysteinyl residue and a S-alkyl-cysteinyl residue, said alkyl being preferably a linear or branched alkyl group, in particular a C1-C3 linear or branched alkyl group, for example a methyl group,
- or Xaa₃ represents a valyl residue and Xaa₁ represents an amino acid residue selected in the group consisting of a methionyl residue, a S-alkyl-homocysteinyl residue and a S-alkyl-cysteinyl residue, said alkyl being preferably a linear or branched alkyl group, in particular a C1-C3 linear or branched alkyl group, for example a methyl group.

In particular embodiments of the invention, in formula (A), Xaa₁ represents a valyl residue, Xaa₂ represents a glycyl residue and Xaa₃ represents a methionyl residue. The elastin-like polypeptide of the invention then contains at least one, preferably several, in particular between 1 and 200, occurrence(s) of a monomeric unit having the amino acid sequence VPGMG (SEQ ID No: 2), which corresponds to the chemical formula (I):

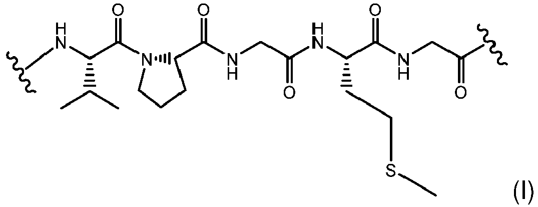

More generally, the elastin-like polypeptide according to the invention preferably comprises a peptide fragment consisting of 1 to 200 repeats of a unit having amino acid sequence:
VPXaa₂XaaG (SEQ ID No: 3)
wherein Xaa₂ denotes a glycyl residue or an alanyl residue, and Xaa denotes a variable amino acid residue that may be any amino acid residue except a prolyl residue, provided that at least one Xaa residue is a sulfur-containing amino acid residue having a side chain of formula -(CH₂)_{z}-S-R', z and R' being as defined above, in particular a methionyl residue.

Due to the presence of at least one, and preferably several, preferably periodically spaced, of such sulfur-containing amino acid residue(s) in the elastin-like polypeptide backbone, the bioconjugate of the invention is highly advantageously both temperature- and singlet-oxygen responsive, which makes it particularly useful for medical applications involving photodynamic therapy treatments.

When the bioconjugate of the invention is used in a photodynamic therapy process, the elastin-like polypeptide acts as a carrier for carrying to a target site, in particular to a tumor site, the photosensitizer unit of the bioconjugate.

No cleavage of the bioconjugate of the invention occurs at the physiological body temperature, and the elastin-like polypeptide ensures a quantitative control on the photosensitizer concentration at the targeted site, in particular by preventing its uncontrolled accumulation and leaking before reaching the targeted site.

The bioconjugate of the invention self-assembles into particles in the body of the treated subject. When photo-irradiation of the photosensitizer unit is carried out, the bioconjugate is oxidized by singlet oxygen produced in the body. More precisely, the hydrophobic thioether group(s) of the one or several sulfur-containing amino acid residue(s), in particular methionyl residue(s), of the bioconjugate, that are prone to oxidation, and therefore highly sensitive to reactive oxygen species, are oxidized into hydrophilic sulfoxide group(s), this oxidation reaction being catalyzed by the photosensitizer unit of the bioconjugate itself.

The invention then takes advantage of the inverse phase transition behavior of the specific elastin-like polypeptides according to the invention. Indeed, these polypeptides present a lower critical solution temperature behavior: below their critical aggregation temperature at a given concentration (cloud point, generally noted as *T*ₜ), their chains are soluble and mostly intrinsically disordered, while hydrophobically collapsing above the *T*ₜ. Due to its sensitivity to oxidation, the thioether group of the sulfur-containing amino acid residue can be readily oxidized into sulfoxide, turning a hydrophobic sulfur-containing amino acid-containing polypeptide with low Tt into a hydrophilic sulfoxide derivative with high *T*ₜ.

After photo-irradiation, it is therefore advantageously observed an increase in the transition temperature of the bioconjugate, to a value far above the physiological body temperature. This increase triggers the disassembly of the bioconjugate particles that had formed in the body of the treated subject. One can hypothesize that the individual bioconjugate molecules released by disassembly of the particles are then capable of diffusing more deeply into the tissues, in particular into a tumor, thereby improving the efficiency of a second photo-irradiation step of the targeted site.

Furthermore, the disassembly of the bioconjugate particles can also be triggered by local hypothermia.

It has also been observed by the inventors that the bioconjugate of the invention remains stable even during long-lasting phototherapies.

In particular embodiments of the invention, the elastin-like polypeptide has the formula (B):
Z-[VPXaa₂Xaa₃G]ₓ-OH (B)
wherein
Z represents a peptide fragment comprising 1 to 20 amino acid residues,
V represents a valyl residue, P represents a prolyl residue, G represents a glycyl residue,
Xaa₂ represents a glycyl residue or an alanyl residue,
and Xaa₃ represents a variable amino acid residue selected from the group consisting of a valyl residue and a sulfur-containing amino acid residue having a side-chain of formula -(CH₂)_{z}-S-R', wherein z and R' are as defined above,
Xaa₃ being such that the molar ratio valyl residue/sulfur-containing amino acid residue in the [VPXaa₂Xaa₃G]ₓ peptide fragment is between 0/1 and 10/1,
and x is an integer between 1 and 200, in particular between 10 and 200 and more particularly between 15 and 80, preferably between 30 and 60, more preferably between 35 and 45 and for example of about 40. In other embodiments of the invention, x is an integer between 20 and 40.

The molar ratio valyl residue/sulfur-containing amino acid residue at the Xaa₃ position in the [VPXaa₂Xaa₃G]ₓ peptide fragment may be between 1/1 and 5/1, more particularly between 2/1 and 4/1. It is for example equal to 3/1.

In particular, in formula (B), Xaa₃ may represent a variable amino acid residue selected from the group consisting of a methionyl residue and a valyl residue, Xaa₃ being such that the molar ratio valyl residue/methionyl residue in the [VPXaa₂Xaa₃G]ₓ peptide fragment is between 0/1 and 10/1, in particular between 1/1 and 5/1, more particularly between 2/1 and 4/1 and for example equal to 3/1. In particular embodiments of the invention, in formula (B), Z denotes a peptide fragment comprising the amino acid sequence MW. This amino acid sequence is then preferably located at the C-terminus of Z.

Z may consist in a peptide fragment having the amino acid sequence MW.

In particular embodiments of the invention, the elastin-like polypeptide has the formula (C):
Z-[VPGVGVPGXaa₃G(VPGVG)₂]_{y} (C)
wherein
Z, V, P, G are as defined above,
Xaa₃ denotes a sulfur-containing amino acid residue having a side-chain of formula -(CH₂)_{z}-S-R', z and R' being as defined above, in particular a methionyl residue,
and y is an integer between 2 and 20, in particular between 5 and 15.

In particular embodiments of the invention, the elastin-like polypeptide has the general chemical formula (Ia): 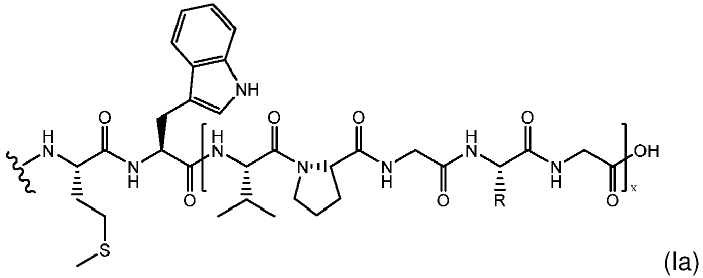 wherein x is an integer between 1 and 200, in particular between 10 and 200 and more particularly between 15 and 80, for example equal to 40, 60 or 80, R represents a variable group chosen from -(CH₂)_{z}-S-R' and -CH(CH₃)₂, and R is such that the molar ratio -CH(CH₃)₂ / -(CH₂)_{z}-S-R' in the elastin-like polypeptide of formula (Ia) is between 0/1 and 10/1, in particular between 1/1 and 5/1, more particularly between 2/1 and 4/1 and for example equal to 3/1.

In particular embodiments of the invention, in formula (Ia), R represents a variable group chosen from -(CH₂)₂-S-CH₃ and -CH(CH₃)₂, and R is such that the molar ratio -CH(CH₃)₂ / -(CH₂)₂-S-CH₃ in the elastin-like polypeptide of formula (Ia) is between 0/1 and 10/1, in particular between 1/1 and 5/1, more particularly between 2/1 and 4/1 and for example equal to 3/1.

In particular embodiments of the invention, the elastin-like polypeptide has an amino acid sequence selected in the group consisting of sequences:
- MGTELAAASEFTHMW[VPGXaa₃G]₂₀ (SEQ ID No: 4),
- MW[VPGVGVPGXaa₃G(VPGVG)₂]₅ (SEQ ID No: 5),
- MW[VPGVGVPGXaa₃G(VPGVG)₂]₁₀ (SEQ ID No: 6),
- MW[VPGVGVPGXaa₃G(VPGVG)₂]₁₅ (SEQ ID No: 7),
- and MW[VPGVGVPGXaasG(VPGVG)₂]₂₀ (SEQ ID No: 8),
wherein Xaa₃ denotes a sulfur-containing amino acid residue having a side-chain of formula -(CH₂)_{z}-S-R', z and R' being as defined above.

The elastin-like polypeptide of the invention may for example have an amino acid sequence selected in the group consisting of sequences:
- MGTELAAASEFTHMW[VPGMG]₂₀ (SEQ ID No: 9),
- MW[VPGVGVPGMG(VPGVG)₂]₅ (SEQ ID No: 10),
- MW[VPGVGVPGMG(VPGVG)₂]₁₀ (SEQ ID No: 11),
- MW[VPGVGVPGMG(VPGVG)₂]₁₅ (SEQ ID No: 12),
- and MW[VPGVGVPGMG(VPGVG)₂]₂₀ (SEQ ID No: 13).

Some of these polypeptides are in particular described in WO-A-2017/021334.

A particularly advantageous elastin-like polypeptide is the polypeptide of amino acid sequence of sequence SEQ ID No: 11 above, having the chemical formula (Ib): 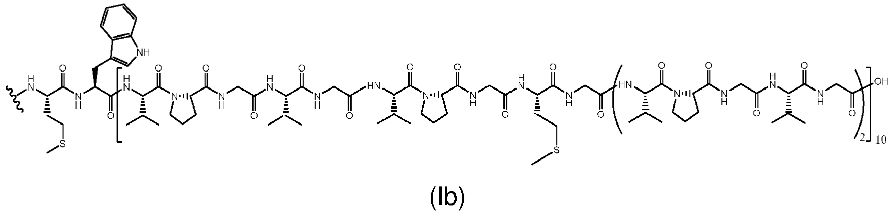 said polypeptide being conjugated to the photosensitizer unit at its N-terminus or at its C-terminus.

In particular embodiments of the invention, the photosensitizer unit is covalently attached to the elastin-like polypeptide by a linker.

A linker is herein defined as a chemical structure capable of covalently binding the photosensitizer unit and the elastin-like polypeptide of the bioconjugate of the invention.

The linker may be a carbonyl group, covalently linked on the one hand to the photosensitizer unit, in particular to an aromatic ring thereof, and on the other end to the N-terminal amine group of the elastin-like polypeptide.

The linker may otherwise have the general formula (II): 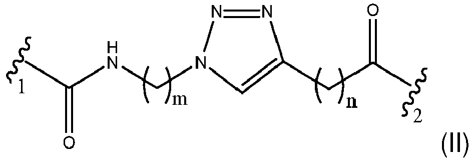 wherein 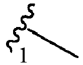
represents a covalent bond to the photosensitizer unit, 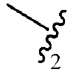
represents a covalent bond to the elastin-like polypeptide,
m is an integer between 1 and 5, preferably equal to 3
and n is an integer between 1 and 6, preferably equal to 2.

The elastin-like polypeptide may be conjugated to the photosensitizer unit at its C-terminal end.

In particular embodiments of the invention, the elastin-like polypeptide is conjugated to the photosensitizer unit at its N-terminal end.

The photosensitizer according to the invention may be methylene blue.

It may otherwise be a boron-dipyrromethene (bodipy)-based photosensitizer. Such compounds, having a core of formula (III), are well-known to the person skilled in the art:

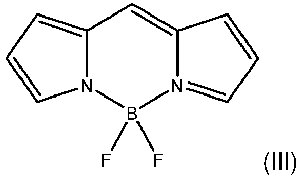

The photosensitizer according to the invention may otherwise be a porphyrin, for example porphin, of formula (IV), or any of the derivatives thereof:

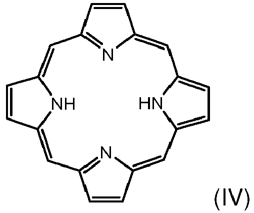

Preferably, the photosensitizer is a phthalocyanine. Phthalocyanines are a group of chemical compounds sharing the structure of formula (V):

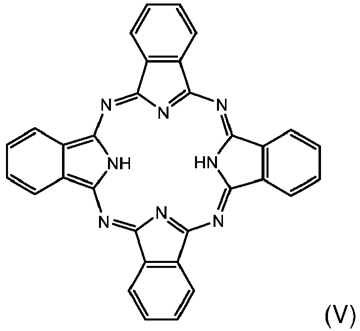

The phthalocyanine unit of the bioconjugate according to the invention is preferably a peripherally-substituted phthalocyanine unit. By "peripherally-substituted" it is herein meant that at least one of the six-membered rings of the phthalocyanine structure is substituted by a substituent other than a hydrogen atom. Preferably, two, three or all four of the six-membered rings of the phthalocyanine structure carry at least one substituent other than a hydrogen atom.

Peripherally-substituted phthalocyanines have in particular the advantages of a higher solubility than unsubstituted phthalocyanines in many solvents, which proves in particular to be useful with regard to the ease of preparation of the bioconjugate of the invention.

The phthalocyanine unit of the bioconjugate of the invention can be axially-substituted or not. An "axially substituted" phthalocyanine unit is defined herein as a phthalocyanine unit carrying substituent(s) located axially relative to the plane of the phthalocyanine structure on a metal or metalloid complexed by the phthalocyanine.

In particular embodiments of the invention, the phthalocyanine unit is a zinc-phthalocyanine unit.

The phthalocyanine unit of the bioconjugate of the invention may have the general formula (V'): 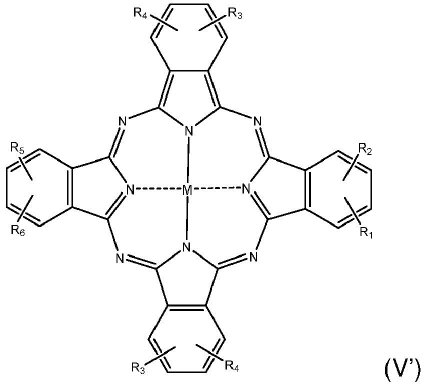 wherein
M represents a metal or metalloid atom,
R₂, R₃, R₄, R₅ and R₆, which may be identical or different, each represent: a hydrogen atom, -COOH, -C≡C-COOH, -CH=CHCOOH, an alkyl group, linear or branched, having from 1 to 12 carbon atoms, -OR₇, -SR₇ or -NR₇R₈, wherein R₇ and R₈, which may be identical or different, each represent a hydrogen atom, an alkyl group, linear or branched, having from 1 to 12 carbon atoms, or a phenyl group optionally substituted by one or more R₉ groups independently selected from the group consisting of an alkyl group, linear or branched, having from 1 to 12 carbon atoms, -OR₁₀, -SR₁₀, and -NR₁₁R₁₂, wherein R₁₀, R₁₁ and R₁₂, which may be identical or different, each represent a hydrogen atom or an alkyl group, linear or branched, having from 1 to 12 carbon atoms,
or one or both pairs R₃ and R₄, and R₅ and R₆, are attached to adjacent carbon atoms and form, together with the ring to which they are attached, an aromatic fused ring system,
and R₁ represents a covalent bond involved in the attachment of said phthalocyanine unit to said elastin-like polypeptide, directly or via a linker.

The general formula (V') above, as well as all other general formulae representing phthalocyanine described herein, encompass all possible regioisomers of the molecule, or mixtures thereof. The term "regioisomers" refers to position isomers having the same functional group or substituent in different positions.

A metalloid herein means a chemical element which is not a metal but which has properties that are in one or more relevant respects comparable to those of metals. Examples of metalloids include silicon and germanium, among others.

In other embodiments of the invention, the phthalocyanine unit may have the free base form of formula (V"): 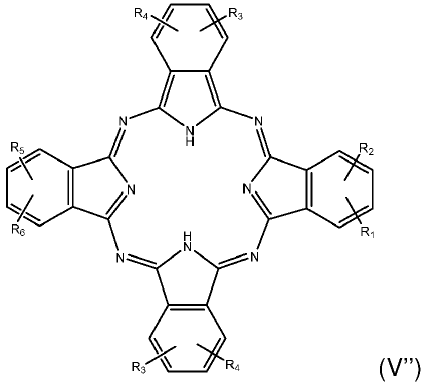 wherein R₁, R₂, R₃, R₄, R₅ and R₆ are as defined above.

The phthalocyanine unit of the bioconjugate of the invention may in particular have the general formula (V'a): 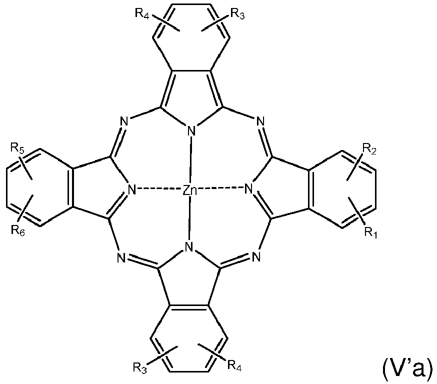 wherein R₁, R₂, R₃, R₄, R₅ and R₆ are as defined above.

In the phthalocyanine unit of formulae (V'), (V") and (V'a), the groups R₁ and R₂ may be located independently in any of the four positions of the corresponding six-membered ring. Similarly, the groups R₃, R₄, R₅ and R₆ may be located independently in any of the four positions of each one of the corresponding six-membered rings of the phthalocyanine unit.

In particularly preferred embodiments of the invention, at least one group, preferably at least two groups, of the R₃, R₄, R₅ and R₆ groups are linear or branched C1-C12 alkyl groups, preferably C4 alkyl groups and more preferably tert-butyl groups.

In particular embodiments, the R₃ and R₅ groups, located independently on any of the four positions of each one of the corresponding six-membered rings, are tert-butyl groups, while the R₄ and R₆ groups are independently a hydrogen atom, a linear or branched alkyl group having from 1 to 12 carbon atoms, -OR₇, -SR₇, or -NR₇R₈, where R₇ and R₈ are as defined above.

Most preferably, the phthalocyanine unit has the general formula (V'b): 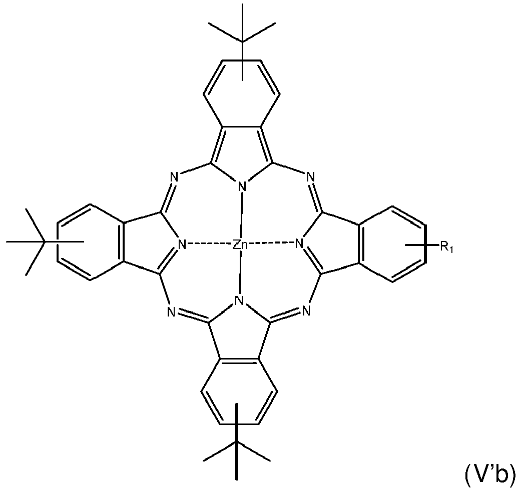 wherein R₁ is as defined above.

In particular embodiments of the invention, the bioconjugate has the general formula (VI): 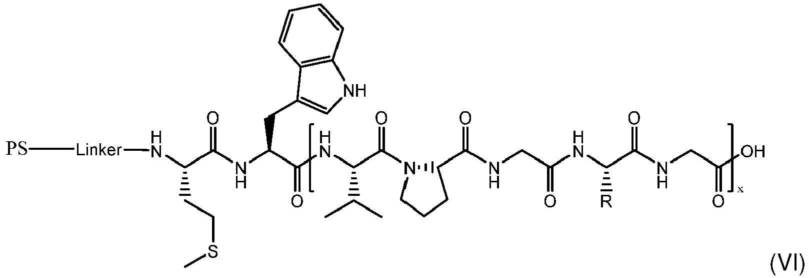
wherein Linker denotes a linker group,
PS denotes a photosensitizer unit,
x is an integer between 1 and 200, in particular between 10 and 200 and more particularly between 15 and 80, for example equal to 40, 60 or 80,
R denotes a variable group chosen from -(CH₂)_{z}-S-R' and -CH(CH₃)₂, z and R' being as defined above, R being such that the molar ratio -CH(CH₃)₂/-(CH₂)_{z}-S-R' in the bioconjugate is between 0/1 and 10/1, in particular between 1/1 and 5/1, more particularly between 2/1 and 4/1 and for example equal to 3/1.

In preferred embodiments of the invention, in formula (VI), R denotes a variable group chosen from -(CH₂)₂-S-CH₃ and -CH(CH₃)₂, R being such that the molar ratio -CH(CH₃)₂/-(CH₂)₂-S-CH₃ in the bioconjugate is between 0/1 and 10/1, in particular between 1/1 and 5/1, more particularly between 2/1 and 4/1 and for example equal to 3/1.

A bioconjugate according to the invention may have the formula (VI'): 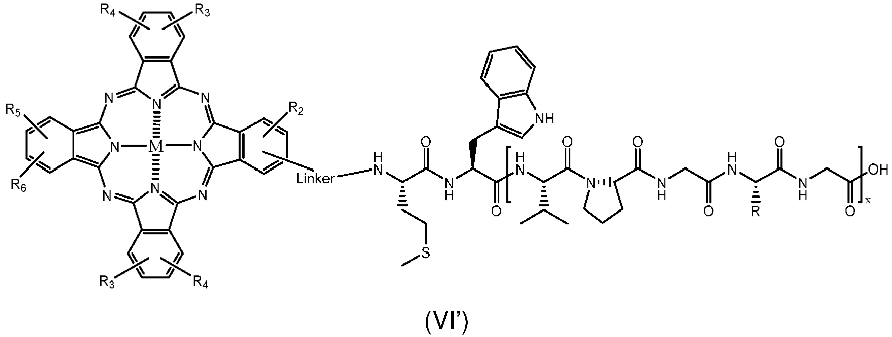
wherein M, R₂, R₃, R₄, R₅ and R₆ are as defined above,
x is an integer between 1 and 200, in particular between 10 and 200 and more particularly between 15 and 80, for example equal to 40, 60 or 80,
and R denotes a variable group chosen from -(CH₂)₂-S-CH₃ and -CH(CH₃)₂, R being such that the molar ratio -CH(CH₃)₂/-(CH₂)₂-S-CH₃ in the bioconjugate is between 0/1 and 10/1, in particular between 1/1 and 5/1, more particularly between 2/1 and 4/1 and for example equal to 3/1.

The bioconjugate of the invention may in particular have the general formula (VI"): 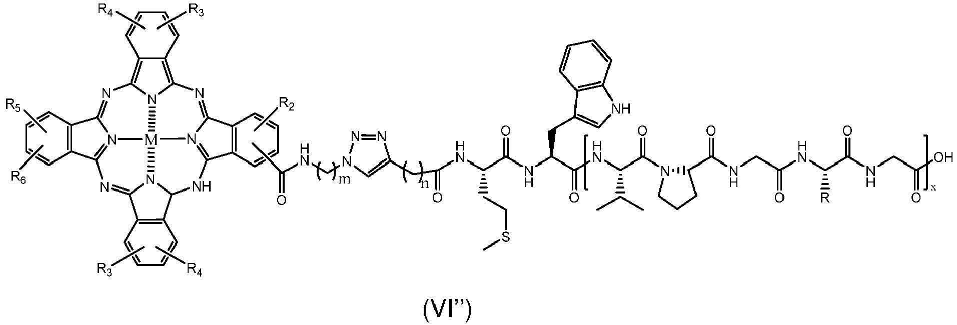 wherein M, R, R₂, R₃, R₄, R₅, R₆, m and n are as defined above, and x is an integer between 1 and 200, in particular between 10 and 200 and more particularly between 15 and 80, for example equal to 40, 60 or 80.

A particularly advantageous bioconjugate according to the invention has the general formula (VI"a): 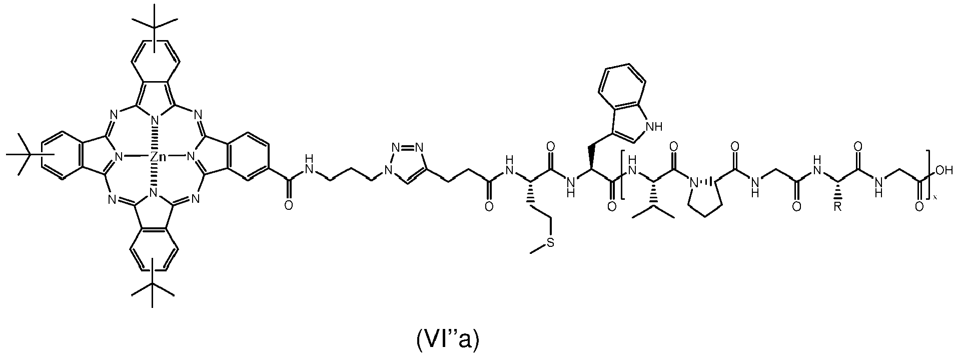 wherein R is as defined above and x is an integer between 1 and 200, in particular between 10 and 200 and more particularly between 15 and 80, for example equal to 40, 60 or 80.

The bioconjugate of the invention may otherwise have formula (Vl'b): 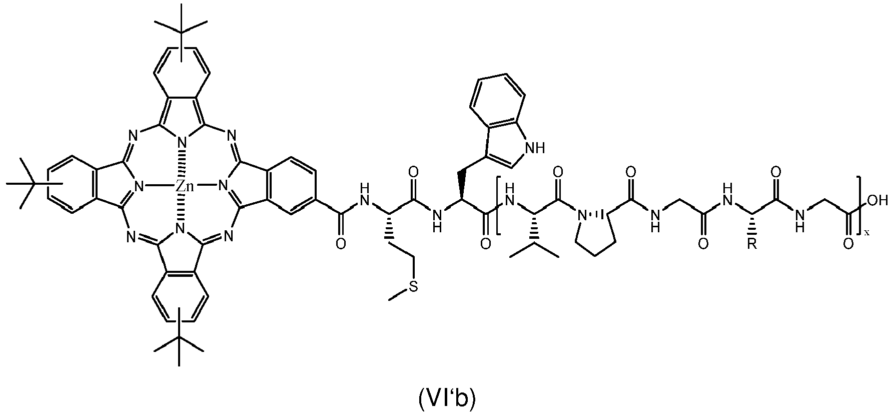 wherein R is as defined above and x is an integer between 1 and 200, in particular between 10 and 200 and more particularly between 15 and 80, for example equal to 40, 60 or 80.

The bioconjugates of formulae (VI"a) and (Vl'b) both have the advantages of being easy to prepare, in a controlled way and at industrial scale.

More generally, the bioconjugates of the invention are non-immunogenic, biocompatible and biodegradable.

They can advantageously be easily produced, with narrow molar mass distribution, in a reproducible way.

The bioconjugates of the invention may be prepared in any way known to person skilled in the art.

Preferably, the photosensitizer unit, for example the phthalocyanine unit, is attached to the elastin-like polypeptide, through a linker, via one or several amide bonds.

A first method for preparing a bioconjugate according to the invention comprises steps of:
- modifying the N-terminal end of an elastin-like polypeptide containing at least one occurrence of a monomeric unit having the formula (A): Xaa₁PXaa₂Xaa₃G (A), wherein P, G, Xaa₁, Xaa₂ and Xaa₃ are as defined herein above, to introduce a terminal alkyne group,
- functionalizing a photosensitizer molecule, in particular a phthalocyanine, preferably a peripherally-substituted phthalocyanine, which is furthermore preferably a zinc-phthalocyanine, to introduce an azide group,
- and realizing a Huisgen's copper(I)-catalyzed alkyne-azide cycloaddition (CuAAC) reaction between the elastin-like polypeptide thus modified and the photosensitizer thus functionalized.

The method according to the invention may further respond to one or more of the features described below, implemented individually or in each of their technically operating combinations.

Elastin-like polypeptides containing at least one occurrence of a monomeric unit having the formula (A), in particular at least one occurrence of the amino acid sequence VPGMG (SEQ ID No: 2), and a method for their preparation and their purification, are known by the person skilled in the art. They are in particular described in WO 2017/021334, together with a method for recombinantly producing them in *Escherichia coli.*

The elastin-like polypeptide implemented in the method of the invention may have any feature or any combination of the features described above in reference to the bioconjugate of the invention, concerning the elastin-like polypeptide part of said bioconjugate.

A particularly preferred elastin-like polypeptide for use in the method of the invention has the formula (B):
Z-[VPXaa₂Xaa₃G]ₓ-OH (B)
wherein
x is an integer between 1 and 200
Z represents a peptide fragment comprising 1 to 20 amino acid residues,
V represents a valyl residue, P represents a prolyl residue, G represents a glycyl residue,
Xaa₂ represents a glycyl residue or an alanyl residue,
and Xaa₃ represents a variable amino acid residue selected from the group consisting of a valyl residue and a sulfur-containing amino acid residue having a side-chain of formula -(CH₂)_{z}-S-R',
Xaa₃ being such that the molar ratio valyl residue/sulfur-containing amino acid residue in the [VPXaa₂Xaa₃G]ₓ peptide fragment is between 0/1 and 10/1.

The elastin-like polypeptide may in particular have the amino acid sequence MW[VPGVGVPGMG(VPGVG)₂]₁₀ (SEQ ID No: 11).

In particular embodiments of the invention, the step of modifying the N-terminal end of the elastin-like polypeptide is carried out by reacting said elastin-like polypeptide with a compound of general formula (VII): 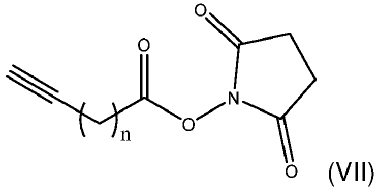 wherein n is an integer between 1 and 6, preferably equal to 2, in such conditions as to form an amide bond involving the terminal nitrogen atom of the elastin-like polypeptide and the carbon atom of the carbonyl group of the compound of general formula (V).

A compound of general formula (VII) particularly useful in the method of the invention is 4-pentynoic acid succinimidyl ester, of formula (Vila):

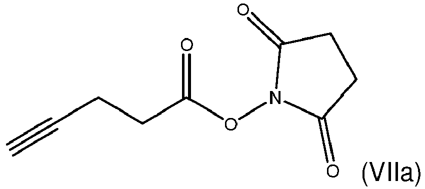

The amidation reaction may be carried out by any method known to the person skilled in the art.

It may for example be carried out according to several, preferably all, of the following features:
- in a polar aprotic solvent, such as dimethylsulfoxide,
- in the presence of a base, such as a tertiary amine, for example N,N-diisopropylethylamine,
- at ambient temperature,
- for a duration of several hours, for example of between 2 and 48 hours, preferably between 6 and 24 hours.

The modified alkyne-(elastin-like polypeptide) thus obtained may be recovered from the reaction medium by any means, for example by dialysis.

The photosensitizer molecule implemented in the method of the invention may have any feature or any combination of the features described above in reference to the bioconjugate of the invention, concerning the photosensitizer unit of the bioconjugate. It is for example a phthalocyanine.

The step of functionalizing said photosensitizer molecule, in particular said phthalocyanine, to introduce an azide group may be carried out in any way known to the person skilled in the art.

In particularly preferred embodiments of the invention, the photosensitizer used as a starting material in the method of the invention is a carboxy-phthalocyanine. "Carboxy-phthalocyanine" means a phthalocyanine such that one of the six-membered rings thereof carries a carboxyl group.

A particularly preferred carboxy-phthalocyanine for use in the method of the invention is a peripherally-substituted carboxy-Zn(II)-phthalocyanine, which preferably has the general formula (VIII): 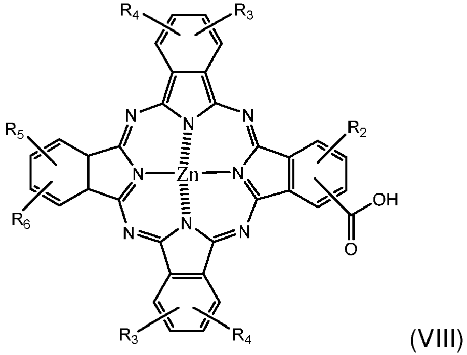 wherein R₂, R₃, R₄, R₅ and R₆ are as defined above.

The phthalocyanine may for example have the formula (Villa):

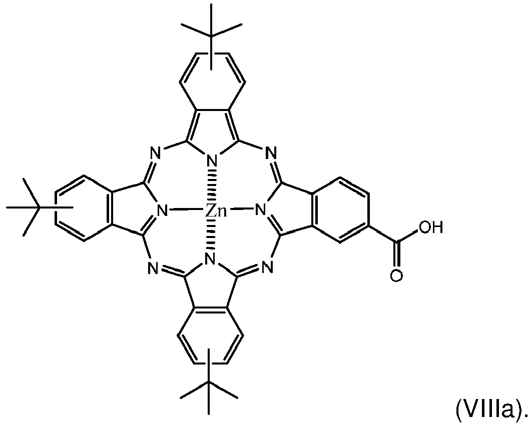

The formulae (VIII) and (Villa) encompass all regioisomers of the molecule, and all mixtures of such regioisomers.

The phthalocyanine of formula (Villa) (9(10),16(17),23(24)-tri-*tert*-butyl-2-carboxy-5,28:14,19-diimino-7,12:21,26-dinitrilo-tetrabenzo[c,h,m,r][1,6,11,16] tetraazacycloeicosinato-(2⁻)-N²⁹,N³⁰,N³¹,N³² zinc(II)) may for example be prepared as described in the publication of Cid et al., Angewandte Chemie International Edition 2007, 46(44), 8358-8362.

It has particularly outstanding photo-physical properties. In particular, it shows in its UV-Visible spectrum a band with an excellent absorption coefficient around 680 nm (Q-band, ε of about 10⁵ M⁻¹.cm⁻¹), which falls in the so-called therapeutical optical window. Singlet oxygen quantum yield of this phthalocyanine, measured in dimethylsulfoxide, mounts up to Φ_{Δ} = 0.72. The presence of the tert-butyl groups not only minimizes the formation of molecular aggregates, but also increases the solubility of the compound in organic solvents.

This phthalocyanine also has the advantage of being easily functionalized, taking advantage of the presence in its structure of the reactive carboxyl function.

The phthalocyanine of formula (Villa) may exist under eight regioisomeric forms. In particular embodiments, the phthalocyanine used as a starting material in the method of the invention is chosen between these eight forms, or any of the mixtures thereof, namely between:
- 9,16,23-tri-*tert*-butyl-2-carboxy-5,28:14,19-diimino-7,12:21,26-dinitrilo-tetrabenzo[c,h,m,r][1,6,11,16]tetraazacycloeicosinato-(2⁻)-N²⁹,N³⁰,N³¹,N³² zinc(II);
- 9,16,24-tri-*tert*-butyl-2-carboxy-5,28:14,19-diimino-7,12:21,26-dinitrilo-tetrabenzo[c,h,m,r][1,6,11,16]tetraazacycloeicosinato-(2⁻)-N²⁹,N³⁰,N³¹,N³² zinc(II);
- 9,17,23-tri-*tert*-butyl-2-carboxy-5,28:14,19-diimino-7,12:21,26-dinitrilo-tetrabenzo[c,h,m,r][1,6,11,16]tetraazacycloeicosinato-(2⁻)-N²⁹,N³⁰,N³¹,N³² zinc(II);
- 9,17,24-tri-*tert*-butyl-2-carboxy-5,28:14,19-diimino-7,12:21,26-dinitrilo-tetrabenzo[c,h,m,r][1,6,11,16]tetraazacycloeicosinato-(2⁻)-N²⁹,N³⁰,N³¹,N³² zinc(II);
- 10,16,23-tri-*tert*-butyl-2-carboxy-5,28:14,19-diimino-7,12:21,26-dinitrilo-tetrabenzo[c,h,m,r][1,6,11,16]tetraazacycloeicosinato-(2⁻)-N²⁹,N³⁰,N³¹,N³² zinc(II);
- 10,16,24-tri-*tert*-butyl-2-carboxy-5,28:14,19-diimino-7,12:21,26-dinitrilo-tetrabenzo[c,h,m,r][1,6,11,16]tetraazacycloeicosinato-(2⁻)-N²⁹,N³⁰,N³¹,N³² zinc(II);
- 10,17,23-tri-*tert*-butyl-2-carboxy-5,28:14,19-diimino-7,12:21,26-dinitrilo-tetrabenzo[c,h,m,r][1,6,11,16]tetraazacycloeicosinato-(2⁻)-N²⁹,N³⁰,N³¹,N³² zinc(II);
- 10,17,24-tri-*tert*-butyl-2-carboxy-5,28:14,19-diimino-7,12:21,26-dinitrilo-tetrabenzo[c,h,m,r][1,6,11,16]tetraazacycloeicosinato-(2⁻)-N²⁹,N³⁰,N³¹,N³² zinc(II);
- and any mixtures thereof.

In particular embodiments of the invention, the step of functionalizing said phthalocyanine is carried out by reacting a carboxy-phthalocyanine with a compound of general formula (IX): 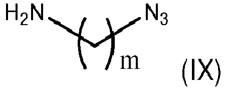
wherein m is an integer between 1 and 5, preferably equal to 3,
in such conditions as to form an amide bond involving the nitrogen atom of the primary amine of the compound of general formula (IX) and the carbon atom of the carboxyl group of the phthalocyanine.

A compound of formula (IX) particularly useful in the method of the invention is 3-azidopropylamine, of formula (IXa):

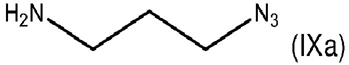

The amidation reaction may be carried out in any way known to the person skilled in the art.

In particular embodiments of the invention the phthalocyanine is reacted with the compound of general formula (IX) according to several, preferably all, of the following features:
- in the presence of a coupling agent such as {[(Z)-(1-Cyano-2-ethoxy-2-oxoethylidene)amino]oxyl-*N,N*-dimethyl(morpholin-4-yl)methaniminium hexafluorophosphate (COMU),
- in the presence of a base, such as a tertiary amine, for example N,N-diisopropylethylamine,
- in a polar aprotic solvent, such as dimethylformamide,
- at ambient temperature,
- for a duration of several hours, for example of between 16 and 28 hours.

The functionalized azide-phthalocyanine thus obtained may be recovered from the reaction medium by any means, for example by extraction with an appropriate solvent, and optionally purified by flash column chromatography. The reaction conditions for the Huisgen's copper(I)-catalyzed alkyne-azide cycloaddition reaction between the modified elastin-like polypeptide and the functionalized photosensitizer thus obtained are classical by themselves.

The reaction may for example be carried out according to several, preferably all, of the following features:
- at ambient temperature,
- in a polar aprotic solvent such as dimethylsulfoxide,
- in the presence of a copper catalytic system such as a mixture of copper sulfate pentahydrate and sodium ascorbate,
- for several hours, for example between 2 and 72 hours.

The bioconjugate thus obtained may be recovered from the reaction medium, and optionally purified, by any means conventional *per se.*

A second method of preparing a bioconjugate according to the invention comprises functionalizing a photosensitizer molecule, in particular a phthalocyanine, preferably containing a free carboxylic acid group, to introduce a N-hydroxysuccinimide-activated ester group, and realizing the direct coupling of said activated group to the N-terminal amine group of an elastin-like polypeptide containing at least one occurrence of a monomeric unit having the formula (A) defined above.

The photosensitizer, in particular the phthalocyanine, and the elastin-like polypeptide used as starting material in this second method may have any feature or any combination of features described above in reference to the first method for preparing a bioconjugate according to the invention.

The functionalizing of the photosensitizer, in particular the phthalocyanine, to introduce a N-hydroxysuccinimide-activated ester group can be carried out by any means known by the person skilled in the art. It is preferably carried out by reacting a phthalocyanine of formula (Villa) with N-Hydroxysuccinimide (NHS) in the presence of N-(3-Dimethylaminopropyl)-N'-ethylcarbodiimide hydrochloride (EDC·HCl), using dichloromethane as solvent, under standard conditions.

The coupling of the thus activated photosensitizer, in particular of the activated phthalocyanine, to the elastin-like polypeptide may be carried out by any method conventional *per se* for reacting a N-hydroxysuccinimide-activated ester with a primary amine in order to form an amide bond.

The reaction may for example be carried out according to several, preferably all, of the following features:
- at ambient temperature,
- in a polar aprotic solvent such as dimethylsulfoxide,
- in the presence of a base such as N,N-diisopropylethylamine,
- for several hours, for example between 2 and 48 hours,
- under an inert atmosphere, for example under a nitrogen N₂ atmosphere.

The bioconjugate thus obtained may be recovered from the reaction medium, and optionally purified, by any means conventional *per se.*

The bioconjugate of the invention proves to be useful for many applications, in particular for medical applications, more particularly as a photosensitizer for photodynamic therapy treatments.

The bioconjugate of the invention can in particular be used for the treatment of a disease by photodynamic therapy, in a subject in need thereof, in particular a mammal, and more particularly a human subject. It can in particular be used for the treatment by photodynamic therapy of a skin disease, of an ocular disease or of any other disease for the treatment of which photodynamic therapy proves relevant. It can for example be used for the treatment of cancer by photodynamic therapy.

The bioconjugate of the invention may be administered to the subject by any administration route that is classical *per se.* It may for example be administered to the subject in need thereof by oral, parenteral, topical, intranasal, rectal or pulmonary route.

Parenteral administration routes, which include subcutaneous, subdural, intravenous, intramuscular, intrathecal, intraperitoneal, intracerebral, intraarterial and intralesional, routes of administration, are particularly preferred in the context of disease, in particular cancer, treatment by photodynamic therapy.

In particularly preferred embodiments, the bioconjugate of the invention is administered to the subject to be treated by injection in a targeted site of the body of said subject, for example in a tumor site.

By way of example, for a use of the bioconjugate of the invention for the treatment of ovarian cancer, the bioconjugate is preferably administered to the subject to be treated by direct injection in the peritoneal cavity.

In the field of cancer treatment, for example of breast or brain cancer treatment, the bioconjugate of the invention may otherwise be administered by topical application after tumor surgery.

The invention can also be expressed in the terms of a method of treating a disease, in particular a skin or an ocular disease, or cancer, in a subject in need thereof by photodynamic therapy, said method comprising:
- administering to said subject a therapeutically-effective amount of the bioconjugate of the invention, in particular by direct injection at a targeted site, for example a tumor site,
- then photo-irradiating said targeted site at an appropriate wavelength for activating the photosensitizer unit, in particular with red light.

By "therapeutically-effective amount" it is herein meant the amount of the bioconjugate that, when administered to the subject for treating the disease, is sufficient to perform such treatment of the disease. The therapeutically-effective amount of the bioconjugate of the invention will depend on several factors, such as the disease and its severity, the age, weight, etc., of the subject to be treated, the particular bioconjugate used, the route and form of administration, etc. The therapeutically-effective amount of the bioconjugate of the invention will be determined by the practitioner for each individual case.

In particularly preferred embodiments of the invention, the method of treating a disease comprises two successive photo-irradiating steps, these steps being carried out a few minutes or hours apart from each other.

In this context of photodynamic therapy, the bioconjugate of the invention advantageously improves the clinical effectiveness of the treatment, compared with phthalocyanines alone or conjugated to different nanocarriers.

The different steps of the method of the invention for treating a disease are illustrated in figure 1, with the example of a cancer as a disease to be treated. The molecules 10 of the bioconjugate of the invention, when administered to a subject, spontaneously self-assemble into particles 11 at the body physiological temperature, which is of about 37 °C for a human subject. The disassembly of these particles accumulated at the tumor site can be remotely triggered by local photo-irradiation with red light 12, and subsequent oxidation of the sulfur-containing amino acid residues by the singlet oxygen 13 produced by the photosensitizer unit of the bioconjugate, which results in an increase of the transition temperature of the bioconjugate. The reactive oxygen species released by the photosensitizer unit bring about the death of cells within the tumor. Simultaneously, the soluble bioconjugate molecules 10 individually released from the particles diffuse deeper into the tumor site and improve the efficacy of a second photo-irradiation step with red light 12. The reactivation of the photosensitizer unit of the bioconjugate, leading to a new step of production of oxygen reactive species such as singlet oxygen 13, may then cause considerable damage to the tumor mass and maximize the therapeutic benefit of the treatment.

Another aspect of the invention is a pharmaceutical composition comprising a bioconjugate according to the invention in a pharmaceutically acceptable vehicle. "Pharmaceutically acceptable" herein means that the vehicle does not exhibit any adverse, allergic or other undesirable effects when administered to a mammal, including humans.

The composition of the invention may be in any form suitable for oral, parenteral, intranasal, sublingual, rectal, transdermal, inhalation or insufflation administration. It is preferably in a form that is suitable for parenteral administration to a subject, in particular to a mammal, more particularly to a human subject, preferably for direct administration to a lesion of a tissue, such as a tumor.

The features and advantages of the invention will emerge more clearly in the light of the following Examples, provided for illustrative purposes only and in no way limitative of the invention.

### Materials

The following reagents are used: sodium chloride (NaCl, VWR, 100%), ammonium acetate (C₂H₃O₂NH₄, Sigma-Aldrich, BioXtra 98%), acetic acid glacial (CH₃CO₂H, Chem-Labs Limited), N,N-diisopropylethylamine (DIPEA, Sigma-Aldrich, 99%), copper (II) sulfate pentahydrate (Cu₂SO₄ 5H₂O, Acros Organics, 98%), (+)-Sodium L-ascorbate (C₆H₇NaO₆, Sigma-Aldrich, 98%), , 3-azidopropylamine (C3H8N4, TCI Europe, >95.0%), COMU^{®} (C₁₂H₁₉F₆N₄O₄P, Sigma-Aldrich, 97%).

The following solvents are used without further purification: ethanol (EtOH, VWR chemicals, analytical grade, 100%), dichloromethane (DCM, Sigma-Aldrich, analytical grade, 99.9%), N, N- dimethylformamide (DMF, Fluka, 99.8%), acetonitrile (ACN, VWR chemicals, HPLC grade, 99.9%), diethyl ether (Et₂O, VWR chemicals, 97%), dimethyl sulfoxide (DMSO, Sigma-Aldrich, 99,9%), 1,4-dioxane (HPLC - Stabilized with BHT, Carlo Erba), n-hexane (99% for analysis, Carlo Erba).

The photosensitizer is a phthalocyanine, herein called TT1, 9(10),16(17),23(24)-Tri-tert-butyl-2-carboxy-5,28:14,19-diimino-7,12:21,26-dinitrilotetrabenzo[c,h,m,r][1,6,11,16]tetraazacycloeicosinato-(2)-N²⁹,N³⁰,N³¹,N³² zinc(II) (mixture of regioisomers).

It has formula (Villa) shown herein above.

The elastin-like polypeptide, herein called ELP, has the chemical formula: 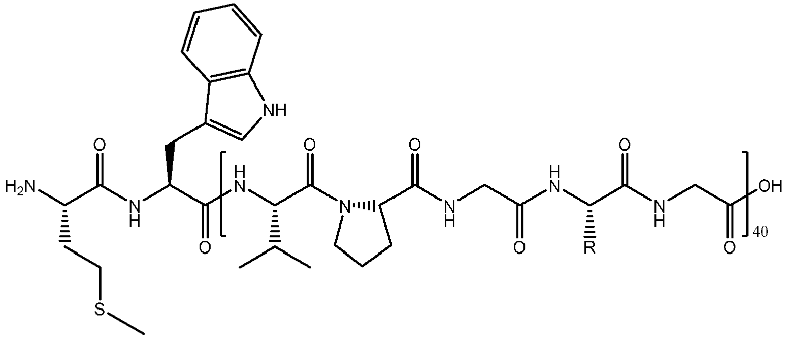
wherein R denotes a variable group chosen from -(CH₂)₂-S-CH₃ and -CH(CH₃)₂, R being such that the molar ratio -CH(CH₃)₂/-(CH₂)₂-S-CH₃ in each unit in square brackets is between equal to 3/1,
and the amino acid sequence SEQ ID No: 11.

### Analysis methods

Nuclear Magnetic Resonance (NMR): ¹H NMR 400 MHz spectra are taken with a Bruker AVANCE^{®} III HD (Liquid-state 400 MHz NMR with 5 mm Prodigy cryoprobe) and Bruker AVANCE^{®} I (Liquid-state 400MHz NMR spectrometer with 5 mm BBFO probe). Deuterated chloroform (CDCl₃, Euriso-top, 99.8%), deuterated dimethylsulfoxide (d-DMSO, Euriso-top, 99.8%) and deuterated water (D₂O, Euriso-top, 99.8%) are used as solvent and reference for the lock.

Size Exclusion Chromatography (SEC): Measurements in DMSO are performed on an UltiMate^{®} 3000 system from Thermo Scientific^{®} equipped with diode array detector DAD. The system also includes a multi-angles light scattering detector MALS and differential refractive index detector dRI from Wyatt technology. Polymers are separated on Tosoh TSK G3000HHR and G2000HHR (7.8*300) columns (exclusion limits from 200 Da to 60 000 Da) at a flowrate of 0.5 mL/min. Columns temperature is held at 80 °C. Dextran from PSS is used as the standard. Dimethylsulfoxide (DMSO + lithium bromide LiBr 1 g/L) is used as the eluent.

Sodium dodecyl sulfate - polyacrylamide gel electrophoresis (SDS-PAGE) analysis: 15 µL of Precision Plus Protein Standards (size marker) and 20 µL from each sample are loaded on the gel trays (BIO-RAD, 4-20% Mini-PROTEAN^{®} TGX Stain-Free^{®} gel). Tris-Glycine-SDS Buffer (TGS 1 x) is used as the loading sample buffer.

Matrix-assisted laser desorption/ionization mass spectrometry (MALDI MS): MALDI-MS spectra are performed by the CESAMO (Bordeaux, France) on an Autoflex maX TOF mass spectrometer (Bruker Daltonics) equipped with a frequency tripled Nd:YAG laser emitting at 355 nm. Spectra are recorded in the linear positive-ion mode with an accelerating voltage of 19 kV. Samples are dissolved in water at 4 mg/ml. The SA matrix (sinapinic acid) solution is prepared by dissolving 10 mg in 1 ml of acetonitrile/0.1% aqueous TFA 50/50. The solutions are combined in a 10:10 volume ratio of matrix to sample. One to two microliters of the obtained solution are deposited onto the sample target and vacuum-dried.

UV-Vis spectroscopy: The transition temperatures (*T*ₜ) are determined by following the turbidity at 600 or 500 nm absorption versus temperature on Agilent Cary 100 UV-Vis spectrophotometer equipped with a multicell thermo-electric temperature controller. Turbidity is measured over a temperature range from 10 to 60 °C at 1°C min⁻¹ of a scan rate before and after the conjugation reaction at three concentrations of phthalocyanine (10, 20 and 30 µM) in phosphate buffer (PBS, pH 7.4).

Dynamic light scattering: The DLS measurements of TT1-ELP (at 20 °C and 37 °C) and TT1-ELP^{PhOx} (at 37 °C) are performed on a Nano ZS 90 instrument (HeNe laser 632.8 nm, Malvern) at a 90° angle at a constant position in the cuvette (constant scattering volume at 30 µM concentration in 1x PBS, pH 7.4). The values of Z-average hydrodynamic diameter (D*_{H}*) and polydispersity index (PDI) are obtained from the 2nd order cumulant fit using a CONTIN-like algorithm.

Atomic Force Microscopy (AFM): Temperature-controlled liquid atomic force microscopy measurements are performed using a Dimension FastScan^{®} Bruker AFM system. The topography images of the bioconjugates are obtained in Peak Force tapping mode, using a Silicon cantilever (ScanAsyst-Fluid+, Bruker) with a typical tip radius of 5 nm. The cantilever resonance is 150 kHz and the spring constant is 0.7 N/m. Samples are prepared by drop-casting a bioconjugate solution of 50 µM onto a freshly cleaved mica or HOPG surface purchased from Agar Scientific, which is directly applied for imaging. AFM imaging process is achieved in the liquid environment at specific temperature. An external heating stage (Bruker) is used to achieve the target temperature at the substrate surface.

### Example 1 - Functionalization of phthalocyanine TT1

TT1 is obtained by the method described in Cid et al., Angewandte Chemie International Edition 2007, 46 (44), 8358-8362.

The functionalization of TT1 to introduce an azide group is carried out according to the synthesis reaction scheme:

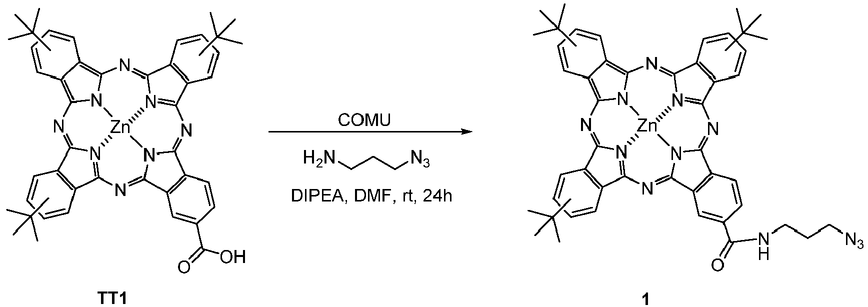

COMU (16.8 mg, 0.039 mmol) is added to a mixture of TT1 (20 mg, 0.025 mmol), freshly distilled DIPEA (5.3 µL) in dry DMF (0.5 mL) at room temperature under argon atmosphere. Then, 3-azidopropylamine (4.0 mg, 0.04 mmol) is added dropwise. The reaction mixture is stirred for 24 h. The mixture is diluted with DCM (2 mL) and extracted with 1N HCl (2 × 5 mL), 1N NaHCO₃ (2 × 5 mL) and saturated NaCl (2 × 5 mL). DCM is then dried with MgSO₄, the solvent is removed, and the crude is directly purified by flash column chromatography on silica gel (dioxane/n-hexane = 50:50) to give 16.4 mg of 1 (yield: 75%) as a dark blue solid.
¹H-NMR (300 MHz, DMSO-*d₆*): δ 9.91 (m, 1H), 9,55-9,30 (m, 8H), 8,71 (m,1H), 8,37 (m, 3H),3,63 (m, 2H), 2.07-2.02 (m, 2H), 1.78 (s, 27H), 1.24 (m, 2H) ppm.
HR-MS (MALDI-TOF, DCTB): Calc. for C₄₈H₄₆N₁₂OZn: [M]+: m/z: 870.3209, found 870.3204.
UV/Vis: (THF): λₘₐₓ (nm) (ε) = 673 (4.70); 607 (4.68), 348 (4.08).

### Example 2 - N-terminal modification of ELP

ELP is obtained and purified as described in Petitdemange et al., Biomacromolecules 2017, 18(2), 544-550.

The modification of ELP to introduce an alkyne group at its N-terminal end is carried out according to the synthesis reaction scheme:

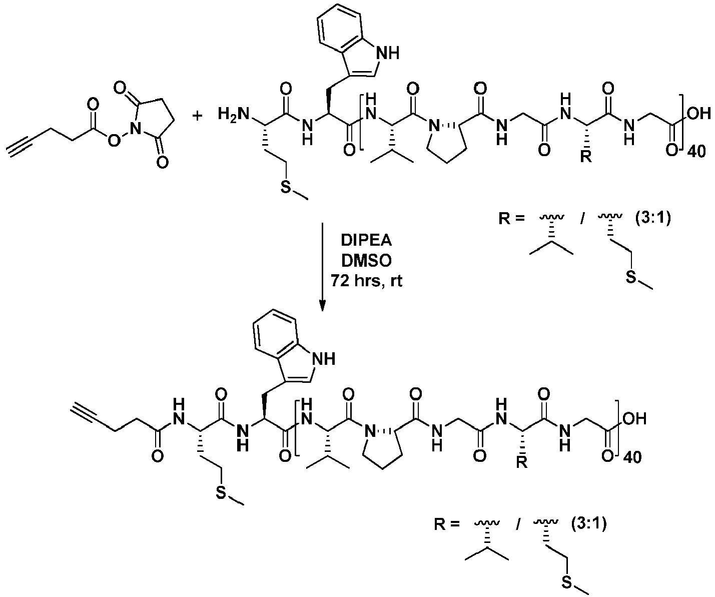

To a solution of ELP (20 mg, 1.17 µmol) in anhydrous DMSO (2 mL) N,N-diisopropylethylamine (1 mg, 1.17 µmol) is added and the solution degassed by nitrogen bubbling. 4-pentynoic acid succinimidyl ester (4 mg, 23.48 µmol) is added into the reaction mixture and stirred 72h under nitrogen at room temperature. Then the solution is diluted with 15 mL H₂O and dialyzed against Milli-Q water in a dialysis bag (cut-off MWCO 3.5 kDa) for 72h. The solution is lyophilized to yield white product of *Alkyne*-ELP (19 mg, 95%).
¹H NMR (400 MHz, D₂O): δ 4.47-4.44 (m, 11H aCH Me), 4.37-4.32 (m, 80H aCH Val, Pro), 4.09- 4.07 (d, 30H aCH Valₓₐₐ), 3.94 - 3.82 (br m, αCH₂ Gly, δCH₂ Pro), 3.64-3.62 (m, δCH₂ Pro), 2.54-2.36 (br m, γCH₂ Met, CH₂CH₂C=CH), 2.23-2.21 (m, βCH₂ Pro), 2.05-1.83 (m, βCH₂ Met, βCH₂ Pro, γCH₂ Pro, βCH₂ Val, εCH₃ Met, CH₂CH₂C=CH), 0.90-0.84 (m, 420 H, γCH₃ Val)

### Example 3 - Synthesis and purification of TT1-ELP bioconjugate

This coupling reaction is carried out according to the synthesis scheme:

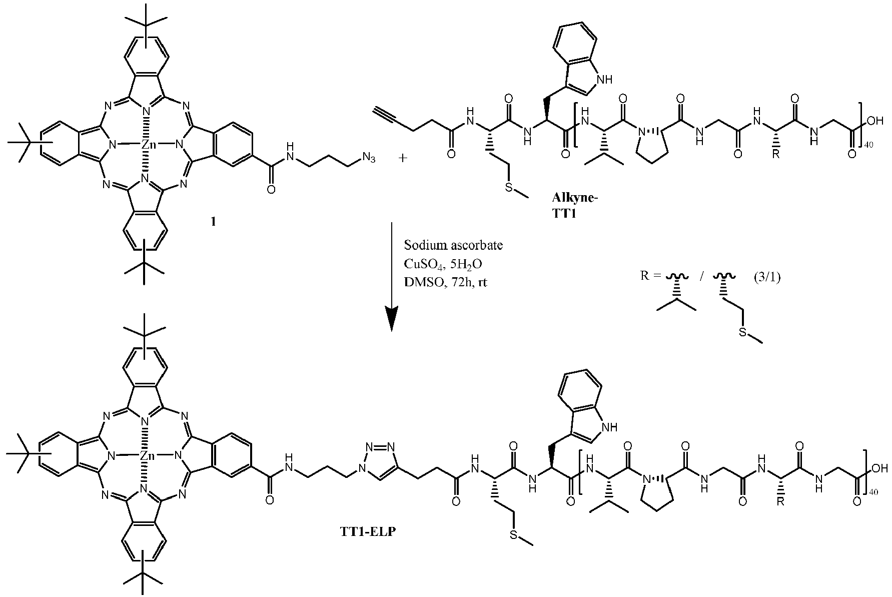

Alkyne-ELP (1 equiv.) and 1 (2 equiv.) are dissolved in dry DMSO (10mg/mL) and C_{U}SO₄,5H₂O (1 equiv.) and sodium ascorbate (2 equiv.) are added and the solution is degassed by freeze-thaw-freeze cycle. The solution is left under stirring for 72 h at room temperature. under nitrogen. Excess cupper is removed by Cuprisorb beads and subsequent filtration. The reaction solution is dialyzed (cut-off MWCO 3.5 kDa) against Milli-Q water for 48 h and lyophilized. The final product is purified by inverse transition cycling (ITC) then lyophilized.

The ITC purification is carried out as follows.

Blue product is dissolved in 5 mL 1M NaCl solution and placed in heating bath (40 °C). Fluctuated bioconjugates are centrifuged 10 min at 38 °C (3800 RPM speed). Supernatant is discarded and the dark blue pellet is dissolved in 3 mL cold water. The solution is centrifuged 30 min at 4 °C (3800 RPM speed) and the pellet is discarded. A few drops of 1 M NaCl solution are added into the supernatant and placed into the heating bath. Aggregated bioconjugates are centrifuged 10 min at 38 °C (3800 RPM speed). Finally, the supernatant is discarded and the pellet is dissolved in cold Milli-Q water and dialyzed against Milli-Q water in a dialysis bag (cut-off MWCO 3.5 kDa) for 24 h to remove the excess salt. The solution is lyophilized to obtain pure blue bioconjugate TT1-ELP.
Yield of pure bioconjugate: 16 mg, 80 %.
¹H NMR (400 MHz, D₂O, 5°C): δ 4.38 (br m, 11H aCH Me), 4.28-4.25 (m, 80H aCH Val, Pro), 4.00- 3.99 (d, 30H aCH Valₓₐₐ), 3.80 - 3.77 (br m, 200H αCH₂ Gly, δCH₂ Pro), 3.54 (br, 40H δCH₂ Pro), 2.47-2.35 (br m, 22H γCH₂ Met), 2.16 (br m, 40H βCH₂ Pro), 1.93-1.80 (br m, 240H βCH₂ Met, βCH₂ Pro, γCH₂ Pro, βCH₂ Val, εCH₃ Met), 0.83-0.70 (m, 420 H, γCH₃ Val).
SEC (DMSO, LiBr 1g/L, standard: Dextran) Mn = 20120 Da, Mw/Mn = 1.129. MALDI MS: theoretical MW= 18032.96 Da, experimental [M+H]⁺=18033.521 Da UV absorption in water λₘₐₓ=337, 633 nm, no fluorescence.

Quantitative modification at the ELP chain end and complete removal of excess TT1 used for the reaction are confirmed by MALDI MS, SDS-PAGE and SEC analyses, as shown in figure 2.

MALDI MS analysis shows a shift of 993.3 Da between the monocharged species, [M+H]⁺, of ELP and TT1-ELP, which is in reasonable agreement with the theoretical value (997.5 Da) (figure 2-A)). SDS-PAGE analysis shows the successful removal of excess TT1 used for the reaction (figure 2-B)). The SEC chromatogram of TT1-ELP (RI detection) reveals a monomodal peak distribution (figure 2-C)). The presence of a small shoulder at shorter retention times is attributed to aggregates of TT1-ELP due to π-π stacking of the TT1 planar backbone. Absorption at 607 nm also indicates the successful attachment of TT1 to the ELP chain end (Figure 2-C)).

### Example 4 - Comparative compound TT1-ELP(O)

In order to study the characteristics of the bioconjugate of the invention after the oxidation of its methionyl residues, a comparative compound TT1-ELP(O), having the same structure as TT1-ELP except that all the sulfur atoms of the 11 methionyl residues are oxidized into sulfoxide form.

To this end, ELP is first reacted with hydrogen peroxide as described in the publication of Petitdemange et al., Biomacromolecules 2017, 18 (2), 544-550, to yield ELP(O) wherein all the sulfur atoms of the methionyl residues have a sulfoxide form.

ELP(O) is then modified at its N-terminal end by the same method as described in Example 1, according the reaction scheme:

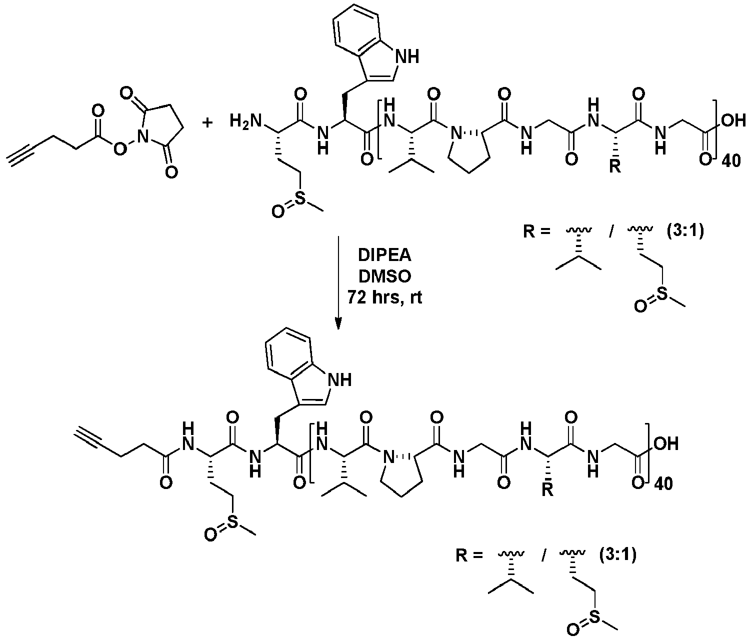

To a solution of ELP(O) (40 mg, 2.32 µmol) in anhydrous DMSO (2 mL) N,N-diisopropylethylamine (0.3 mg, 2.32 µmol) is added and followed by addition of 4-pentynoic acid succinimidyl ester (9 mg, 46.48 µmol). The reaction mixture is stirred for 72h under argon at room temperature. Then the solution is diluted with 15 mL H₂O and dialyzed against Milli-Q water in a dialysis bag (cut-off MWCO 3.5 kDa) for 72h. The solution is lyophilized to yield white product *Alkyne*-ELP(O) (30 mg, 75 %).
¹H NMR (400 MHz, D₂O, 25° C): δ 4.29-4.23 (m, 80 H, aCH Val, Pro), 4.09-3.99 (d, 30 H, aCH VPGVG), 2.85-2.73 (m, 22 H, CH₂S Met), 2.38-2.29 (br m, γCH₂ Met, CH₂CH₂C≡CH), 2.56 (s, 33 H, SCHs Met), 0.82-0.75 (br m, 420 H, CH₃ Val).

¹H NMR analysis confirms the quantitative oxidation of ELP as well as the complete functionalization of the *N*-terminal chain end.

TT1-azide conjugation to *alkyne-ELP(O)* is carried out as described in Example 3 and confirmed by MALDI MS, SDS-PAGE, SEC, NMR, and UV-Vis spectroscopy analyses, as shown in figure 2.

MALDI MS analysis shows a shift of 1,174 Da between the monocharged species, [M+H]⁺, of ELP and TT1-ELP(O), in good agreement with the theoretical value (1,177 Da) (figure 2-A)). The SEC chromatogram of TT1-ELP(O) (RI detection) shows a narrow monomodal peak distribution which overlaps with the UV detector signal at 607 nm indicating successful conjugation of TT1 (figure 2-D)).

Optical characterization of TT1-ELP and TT1-ELP(O) bioconjugates at ambient temperature shows no difference in absorption maxima at PBS buffer (pH 7.4, λ_{abs}=337 and 633 nm) (figure 2-E)). Owing to the conjugation to the polypeptide, aqueous environment with higher ionic strength and stacking of TT1 chain ends, the absorption peaks of the bioconjugates get broader and blue-shifted from 673 nm (TT1 in THF) to 633 nm (conjugate in PBS).

### Example 5 - Photo-irradiation of TT1-ELP

A solution of the bioconjugate TT1-ELP according to the invention in D₂O is subjected to irradiation with a red-light emitting diode. ¹H NMR spectroscopy is used to follow the selective oxidation of methionyl residues.

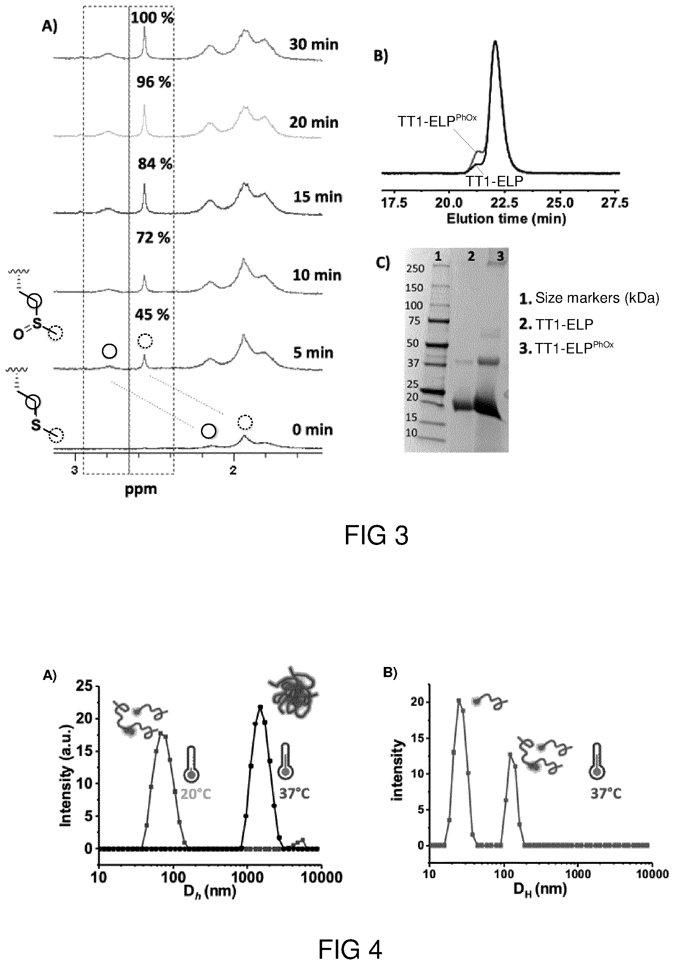

3 mg of TT1-ELP are dissolved in 0.5 mL of D₂O and transferred to the NMR tube. ¹H NMR spectra is taken before and after every 5 min during 30 min of the LED (Luzchem LEDi, red region 630-640 nm, irradiance > 2100 W/m²) irradiation to monitor the photooxidation of the methionyl residue at ambient temperature *via* 200 MHz ¹H NMR. The resulting bioconjugate is herein called TT1-ELP^{PhOx}. The formation of sulfoxide during the red emitting LED irradiation over 5, 10, 15, 20 and 30 minutes is monitored by the shift of the resonance peak at 2.1 ppm attributed to the protons of the methyl group adjacent to the sulfur in the thioether form (-SCH₃) to 2.6 ppm in the sulfoxide form (-S(O)CH₃), observed in figure 3A). Approximately 50 % oxidation was observed in 5 min, while 30 min irradiation caused complete oxidation of the 11 methionyl residues of the ELP.

The overlapped SEC chromatograms of TT1-ELP and TT1 -ELP^{PhOx} show narrow monomodal peak distribution confirming the selectivity of the oxidation process without any traces of crosslinking or degradation in the backbone (figure 3-B)). Increased dimer population displayed in SEC and also observed in SDS-PAGE gel (figure 3-C)) is probably due to the strong hydrophobic interactions between TT1 chain ends.

### Example 6 - Onset transition temperatures

The thermo-responsive character of ELP, before and after TT1 conjugation, is measured by turbidity assays in phosphate buffer (PBS, pH 7.4) following the absorbance at 600 and 500 nm, respectively, over a temperature range from 10 to 60 °C. Measurements are performed at three concentrations (10, 20 and 30 µM) at which TT1-ELP is fully soluble at 10 °C.

The same analysis is carried out for TT1-ELP(O) (obtained by chemical oxidation of methionyl groups) and TT1-ELP^{PhOx} (obtained by oxidation triggered by photo-irradiation of methionyl groups).

The results are shown in Table 1.

**Table 1 - Onset transition temperatures of ELP and different bioconjugates at different concentrations in PBS**

| Compound | *T*ₜ in PBS (°C) | | |
|---|---|---|---|
| | 10 µM | 20 µM | 30 µM |
| ELP | 38 | 36 | 35 |
| TT1-ELP | 29 | 28 | 27 |
| TT1-ELP(O) | 45.9 | 45.6 | 44.8 |
| TT1-ELP^{PhOx} | 44.9 | 44.7 | 44.2 |

At the concentrations studied, the transition temperature (Tt) of ELP is in the range of 35-38 °C, close to physiological body temperature. After conjugation of TT1, the Tt is found to drop by approximately 10 °C, the Tt of TT1-ELP ranging from 29 °C at 10 µM to 27 °C at 30 µM. This was expected due to the strong hydrophobic character of TT1.

Dynamic light scattering evidences the formation of stable particles of 1.5 µm above the *T*ₜ of TT1-ELP suggesting that TT1-ELP shall be self-assembled when used *in vivo.*

In clinical application, these smart self-assembled particles could be disassembled upon local cryothermia at the tumor to let the TT1-ELP bioconjugate diffuse deeper into the tumor mass before applying the photodynamic treatment.

Interestingly, the disassembly of TT1-ELP particles could also be triggered by oxidation of methionyl residues by ¹O₂ generated upon TT1 activation by near infrared light. Indeed, the thermo-responsive behavior of the chemically oxidized conjugate TT1-ELP(O) measured in similar conditions to those used for TT1-ELP shows a huge jump (of about 17°C) in Tt as compared to the non-oxidized bioconjugate. With a *T*ₜ of about 45°C, well above the body temperature, the oxidized bioconjugate TT1-ELP(O) shall therefore be soluble at 37°C.

The thermo-responsive character of the photo-oxidized bioconjugate, TT1-ELP^{PhOx}, is compared to the one of the starting TT1-ELP bioconjugate and of the chemically oxidized reference compound TT1-ELP(O). The Tt at 30 µM in PBS is shifted from 35 °C for TT1-ELP to 44 °C, in perfect concordance with the value obtained for the chemically synthesized sulfoxide version TT1-ELP(O)₁. The drastic change in physicochemical properties of the ELP backbone resulting from the sulfoxide formation, shifting the phase transition temperature of the conjugate, shall cause particles destabilization and disassembly.

### Example 7 - Size distribution analysis

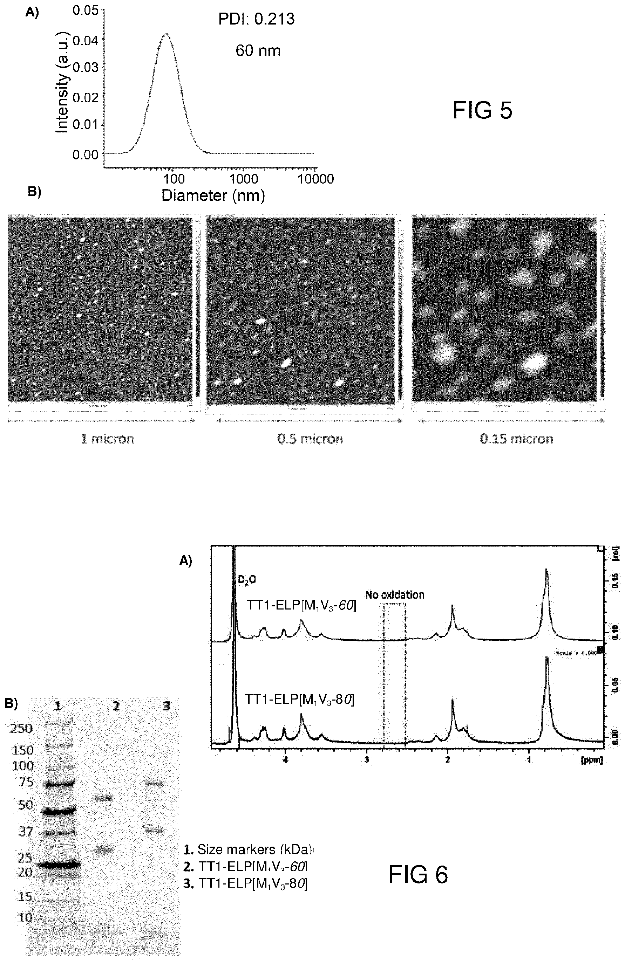

In order to mimic the self-assembly and photooxidation-triggered disassembly profile of TT1-ELP, dynamic light scattering (DLS) measurements are performed in physiological conditions (PBS pH 7.4 at 37 °C) on TT1-ELP and TT1-ELP^{PhOx}. The results are shown on figure 4.
Increase of the solution temperature to 37 °C results in the formation of large particles (1.5 µm hydrodynamic diameter) in PBS (pH 7.4), while dropping the temperature back to 20 °C leads to the disassembly of these aggregates, as shown in figure 4-A).
In contrast, DLS analysis of TT1-ELP^{PhOx} at 37 °C in PBS (pH 7.4) evidences the presence of single chains or small size dimers/multimers while no aggregate around 1.5 µm is detected, as shown in figure 4-B)).
Due to the stacking tendency of TT1 upon direct dissolution of TT1-ELP in PBS at ambient temperature, 60 nm micelles with narrow size distribution (PDI 0.2) are observed in DLS measurement conducted at 165° angle *via* 657 nm laser (figure 5-A)). Temperature-controlled high-speed AFM imaging also confirms the presence of the micelles even at 15 °C, as shown in figure 5-B).

The above results show that upon specific photo-irradiation of TT1, ELP is oxidized into its sulfoxide derivative ELP(O), turning the hydrophobic TT1-ELP bioconjugate according to the invention, self-assembled into particles at physiological body temperature, into a hydrophilic, soluble TT1-ELP(O) derivative.
This experimental demonstration of the multi-responsive behavior of the bioconjugate of the invention proves very interesting in terms of clinical applications. Indeed, in the context of photodynamic therapy, self-assembled particles of TT1-ELP accumulated at the tumor site disassemble upon local photoirradiation due to methionyl residues oxidation and increase of *T*ₜ. The soluble photo-oxidized bioconjugate molecules shall then diffuse more easily and deeper into the tumor tissue, including in dense tumors, and allow a second photo-irradiation step to be carried out. The reactivation of TT1 may then cause considerable damage to the tumor mass.

### Example 8 - Alternative method of preparing a bioconjugate according to the invention

### Step 1: Activation of phthalocyanine TT1

TT1 is obtained by the method described in Cid et al., Angewandte Chemie International Edition 2007, 46 (44), 8358-8362.

The functionalization of TT1 to introduce a N-hydroxysuccinimide-activated ester is carried out according to the synthesis reaction scheme:

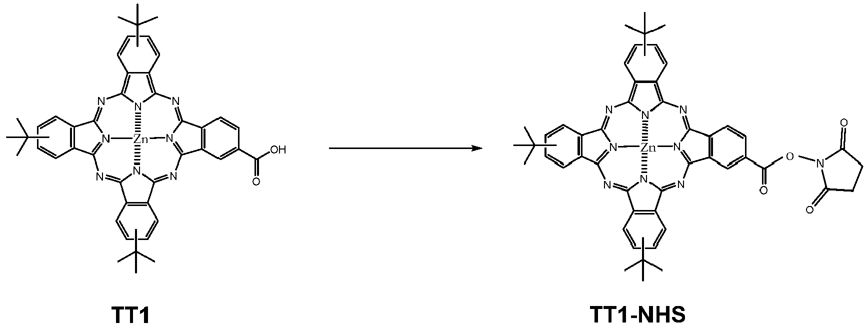

For this activation reaction, TT1 is reacted with N-Hydroxysuccinimide (NHS) in the presence of N-(3-Dimethylaminopropyl)-N'-ethylcarbodiimide hydrochloride (EDC·HCl), using dichloromethane as solvent, under inert atmosphere at room temperature, under standard conditions. The solution is diluted with dichloromethane and washed with H₂O. The organic phase is dried with Na₂SO₄ and the solvent is removed under reduced pressure. The resulting crude is purified by column chromatography on silica gel (dioxane:hexane, 1:1) to afford the functionalized TT1, called TT1-NHS (80 %) as a dark green solid.

### Step 2: Synthesis and purification of TT1-ELP bioconjugates

Two ELP are used in this experiment:
ELP[M₁V₃-60] having the formula: 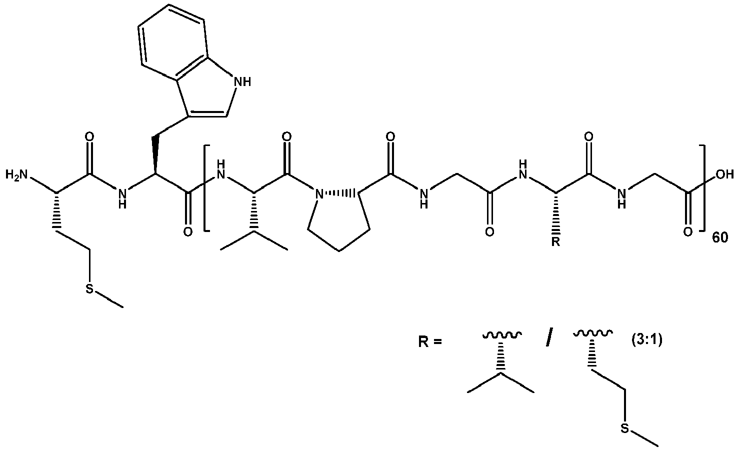
and ELP[M₁V₃-80] having the formula:

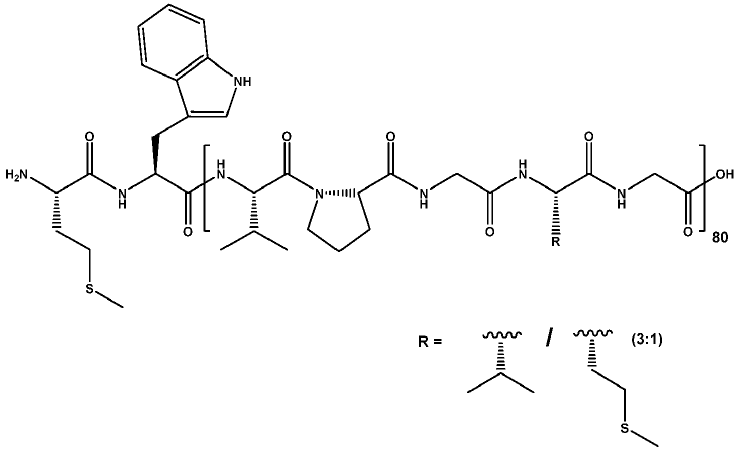

This coupling reactions are carried out according to the following synthesis scheme:

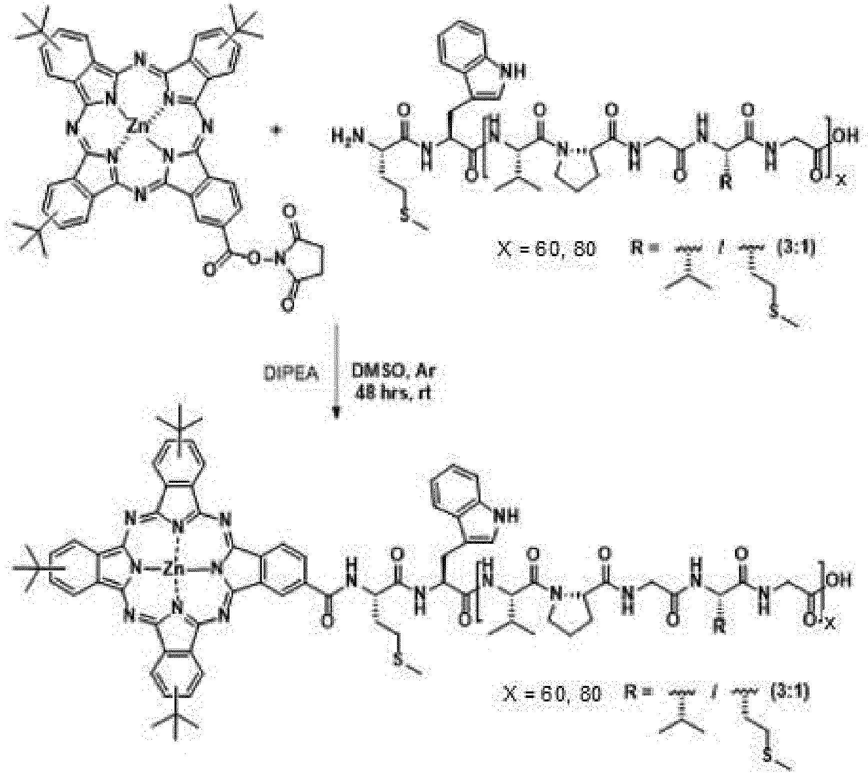

To a solution of ELP[MiVs-*60*]/ ELP[M₁V₃-*80*] (100 mg, 1 equiv.) and TT1-NHS (1.2 equiv.) in anhydrous DMSO (25 mg.mL⁻¹ concentration), N,N-diisopropylethylamine (1 equiv.) is added and the solution is degassed by bubbling argon for 5 min. The reaction mixture is stirred 48 h under N₂ at room temperature. Then the solution is precipitated in diethyl ether. The precipitate is dissolved in 3 mL water and purified on a Sephadex^{®} 100G column. The collected sample is lyophilized and additionally purified *via* inverse transition cycling (ITC). The TT1-ELP bioconjugate pellet is dissolved in 20 mL H₂O and dialyzed against ultrapure water in a dialysis bag (molecular weight cut-off 15 kDa) for 48h and lyophilized.
TT1-ELP[M₁V₃-*60*]: 101 mg, 3.85 µmol, 98%
TT1-ELP[M₁V₃-*80*]: 96 mg, 2.84 µmol, 94%

Results of the characterization of these bioconjugates are shown in figure 6 (¹H NMR spectra in D₂O at 20 °C, in figure 6-A), SDS-PAGE analysis, in figure 2-B)). These results demonstrate the successful synthesis of the bioconjugates according to the invention.

## Claims

1. A bioconjugate comprising a photosensitizer unit and an elastin-like polypeptide conjugated thereto, said elastin-like polypeptide containing at least one occurrence of a monomeric unit having the formula (A):
Xaa₁PXaa₂Xaa₃G (A)
wherein P represents a prolyl residue, G represents a glycyl residue, Xaa₂ represents a glycyl residue or an alanyl residue and:
- either Xaa₁ represents a valyl residue and Xaa₃ represents a sulfur-containing amino acid residue having a side chain of formula -(CH₂)_{z}-S-R', wherein z is equal to 1 or 2 and R' represents a linear, branched and/or cyclic C1-C22 hydrocarbon radical, saturated and/or unsaturated, aromatic or not, optionally substituted, optionally interrupted by one or more heteroatoms and/or by one or more groups comprising at least one heteroatom or group comprising at least one heteroatom,
- or Xaa₃ represents a valyl residue and Xaa₁ represents a sulfur-containing amino acid residue having a side chain of formula -(CH₂)_{z}-S-R', wherein z is equal to 1 or 2 and R' is as defined above.

2. A bioconjugate according to claim 1, wherein R' represents a C1-C3 alkyl group.

3. A bioconjugate according to claim 1 or 2, wherein, in formula (A):
- either Xaa₁ represents a valyl residue and Xaa₃ represents an amino acid residue selected in the group consisting of a methionyl residue, a S-alkyl-homocysteinyl residue and a S-alkyl-cysteinyl residue,
- or Xaa₃ represents a valyl residue and Xaa₁ represents an amino acid residue selected in the group consisting of a methionyl residue, a S-alkyl-homocysteinyl residue and a S-alkyl-cysteinyl residue,

4. A bioconjugate according to any of claims 1 to 3, wherein said photosensitizer is selected in the group consisting of phthalocyanines, porphyrins, bodipy-based photosensitizers and methylene blue.

5. A bioconjugate according to any of claims 1 to 4, wherein said photosensitizer unit is covalently attached to said elastin-like polypeptide by a linker.

6. A bioconjugate according to any of claims 1 or 5, wherein said elastin-like polypeptide is conjugated to said photosensitizer unit at its N-terminal end.

7. A bioconjugate according to any of claims 1 to 6, wherein said elastin-like polypeptide has the formula (B):
Z-[VPXaa₂Xaa₃G]ₓ-OH (B)
wherein
x is an integer between 1 and 200
Z represents a peptide fragment comprising 1 to 20 amino acid residues,
V represents a valyl residue, P represents a prolyl residue, G represents a glycyl residue,
Xaa₂ represents a glycyl residue or an alanyl residue,
and Xaa₃ represents a variable amino acid residue selected from the group consisting of a valyl residue and a sulfur-containing amino acid residue having a side-chain of formula -(CH₂)_{z}-S-R', z and R' being as defined in claim 1 or 2,
Xaa₃ being such that the molar ratio valyl residue/sulfur-containing amino acid residue in the [VPXaa₂Xaa₃G]ₓ peptide fragment is between 0/1 and 10/1.

8. A bioconjugate according to claim 7, wherein, in the formula (B), Z represents a peptide fragment having the amino acid sequence MW.

9. A bioconjugate according to any of claims 1 to 8, wherein said elastin-like polypeptide has an amino acid sequence selected in the group consisting of sequences: MGTELAAASEFTHMW[VPGMG]₂₀ (SEQ ID No: 9), MW[VPGVGVPGMG(VPGVG)₂]₅ (SEQ ID No: 10), MW[VPGVGVPGMG(VPGVG)₂]₁₀ (SEQ ID No: 11), MW[VPGVGVPGMG(VPGVG)₂]₁₅ (SEQ ID No: 12) and MW[VPGVGVPGMG(VPGVG)₂]₂₀ (SEQ ID No: 13).

10. A bioconjugate according to any of claims 1 to 9, wherein said photosensitizer unit is a phthalocyanine unit having the general formula (V'): 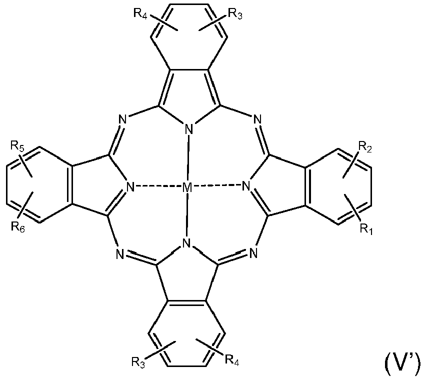 wherein
M represents a metal or metalloid atom,
R₂, R₃, R₄, R₅ and R₆, which may be identical or different, each represent: a hydrogen atom, -COOH, -C≡C-COOH, -CH=CHCOOH, an alkyl group, linear or branched, having from 1 to 12 carbon atoms, -OR₇, -SR₇ or -NR₇R₈, wherein R₇ and R₈, which may be identical or different, each represent a hydrogen atom, an alkyl group, linear or branched, having from 1 to 12 carbon atoms, or a phenyl group optionally substituted by one or more R₉ groups independently selected from the group consisting of an alkyl group, linear or branched, having from 1 to 12 carbon atoms, -OR₁₀, -SR₁₀, and -NR₁₁R₁₂, wherein R₁₀, R₁₁ and R₁₂, which may be identical or different, each represent a hydrogen atom or an alkyl group, linear or branched, having from 1 to 12 carbon atoms,
or one or both pairs R₃ and R₄, and R₅ and R₆, are attached to adjacent carbon atoms and form, together with the ring to which they are attached, an aromatic fused ring system,
and R₁ represents a covalent bond involved in the attachment of said phthalocyanine unit to said elastin-like polypeptide.

11. A method of preparing a bioconjugate according to any of claims 1 to 10, **characterized in that** it comprises:
- modifying the N-terminal end of an elastin-like polypeptide containing at least one occurrence of a monomeric unit having the formula (A): Xaa₁PXaa₂Xaa₃G (A), wherein P, G, Xaa₁, Xaa₂ and Xaa₃ are as defined in any of claims 1 to 3, to introduce a terminal alkyne group,
- functionalizing a photosensitizer molecule to introduce an azide group,
- and realizing a Huisgen's copper(I)-catalyzed alkyne-azide cycloaddition reaction between the elastin-like polypeptide so modified and the photosensitizer so functionalized.

12. A bioconjugate according to any of claims 1 to 10 for its use for the treatment of a disease, in particular for the treatment of a skin disease or an ocular disease, by photodynamic therapy.

13. A bioconjugate for its use as claimed in claim 12, wherein said bioconjugate is administered by injection in a targeted site of the body of a subject to be treated.

14. A pharmaceutical composition comprising a bioconjugate according to any of claims 1 to 10 in a pharmaceutically acceptable vehicle.

15. A pharmaceutical composition according to claim 14, in a form that is suitable for parenteral administration to a subject.


SEQUENCE LISTING

<110>  UNIVERSITE DE BORDEAUX
       INSTITUT POLYTECHNIQUE DE BORDEAUX
       CENTRE NATIONAL DE LA RECHERCHE SCIENTIFIQUE
       UNIVERSIDAD AUTONOMA DE MADRID

<120>  BIOCONJUGATE COMPRISING A PHOTOSENSITIZER UNIT, METHOD FOR ITS
       PREPARATION AND USE THEREOF

<130>  34281 EP

<160>  13

<170>  PatentIn version 3.5

<210>  1
<211>  5
<212>  PRT
<213>  Artificial sequence

<220>
<223>  Monomeric unit of elastin-like polypeptide


<220>
<221>  MISC_FEATURE
<222>  (1)..(1)
<223>  Xaa denotes a variable amino acid residue that may be any natural
       or non-natural amino acid residue except a prolyl residue or a
       derivative thereof, in particular a valine residue

<220>
<221>  MISC_FEATURE
<222>  (3)..(3)
<223>  Xaa is glycine or alanine

<220>
<221>  MISC_FEATURE
<222>  (4)..(4)
<223>  Xaa denotes a variable amino acid residue that may be any natural
       or non-natural amino acid residue except a prolyl residue or a
       derivative thereof, in particular a valine residue

<400>  1

Xaa Pro Xaa Xaa Gly
1               5


<210>  2
<211>  5
<212>  PRT
<213>  Artificial sequence

<220>
<223>  Monomeric unit of elastin-like polypeptide

<400>  2

Val Pro Gly Met Gly
1               5

<210>  3
<211>  5
<212>  PRT
<213>  Artificial sequence

<220>
<223>  Monomeric unit of elastin-like polypeptide



<220>
<221>  MISC_FEATURE
<222>  (3)..(3)
<223>  Xaa is glycine or alanine

<220>
<221>  MISC_FEATURE
<222>  (4)..(4)
<223>  Xaa denotes a variable amino acid residue that may be any amino
       acid except proline, in particular a sulfur-containing amino acid
       residue having a side chain of formula -(CH2)z-S-R′ wherein z is
       1 or 2 and R′ is a C1-C22 hydrocarbon radical

<400>  3

Val Pro Xaa Xaa Gly
1               5


<210>  4
<211>  115
<212>  PRT
<213>  Artificial sequence

<220>
<223>  Elastin-like polypeptide



<220>
<221>  MISC_FEATURE
<222>  (19)..(114)
<223>  Xaa denotes a sulfur-containing amino acid residue having a
       side-chain of formula -(CH2)z-S-R′ wherein z is 1 or 2 and R′ is
       a C1-C22 hydrocarbon radical

<400>  4

Met Gly Thr Glu Leu Ala Ala Ala Ser Glu Phe Thr His Met Trp Val
1               5                   10                  15


Pro Gly Xaa Gly Val Pro Gly Xaa Gly Val Pro Gly Xaa Gly Val Pro
            20                  25                  30


Gly Xaa Gly Val Pro Gly Xaa Gly Val Pro Gly Xaa Gly Val Pro Gly
        35                  40                  45


Xaa Gly Val Pro Gly Xaa Gly Val Pro Gly Xaa Gly Val Pro Gly Xaa
    50                  55                  60

Gly Val Pro Gly Xaa Gly Val Pro Gly Xaa Gly Val Pro Gly Xaa Gly
65                  70                  75                  80

Val Pro Gly Xaa Gly Val Pro Gly Xaa Gly Val Pro Gly Xaa Gly Val
                85                  90                  95

Pro Gly Xaa Gly Val Pro Gly Xaa Gly Val Pro Gly Xaa Gly Val Pro
            100                 105                 110

Gly Xaa Gly
        115

<210>  5
<211>  102
<212>  PRT
<213>  Artificial sequence

<220>
<223>  Elastin-like polypeptide

<220>
<221>  MISC_FEATURE
<222>  (11)..(91)
<223>  Xaa denotes a sulfur-containing amino acid residue having a
       side-chain of formula -(CH2)z-S-R' wherein z is 1 or 2 and R' is
       a C1-C22 hydrocarbon radical

<400>  5

Met Trp Val Pro Gly Val Gly Val Pro Gly Xaa Gly Val Pro Gly Val
1               5                   10                  15

Gly Val Pro Gly Val Gly Val Pro Gly Val Gly Val Pro Gly Xaa Gly
            20                  25                  30

Val Pro Gly Val Gly Val Pro Gly Val Gly Val Pro Gly Val Gly Val
        35                  40                  45

Pro Gly Xaa Gly Val Pro Gly Val Gly Val Pro Gly Val Gly Val Pro
    50                  55                  60

Gly Val Gly Val Pro Gly Xaa Gly Val Pro Gly Val Gly Val Pro Gly
65                  70                  75                  80

Val Gly Val Pro Gly Val Gly Val Pro Gly Xaa Gly Val Pro Gly Val
                85                  90                  95

Gly Val Pro Gly Val Gly
            100

<210>  6

<211>  202
<212>  PRT
<213>  Artificial sequence

<220>
<223>  Elastin-like polypeptide

<220>
<221>  MISC_FEATURE
<222>  (11)..(191)
<223>  Xaa denotes a sulfur-containing amino acid residue having a
       side-chain of formula -(CH2)z-S-R' wherein z is 1 or 2 and R' is
       a C1-C22 hydrocarbon radical

<400>  6

Met Trp Val Pro Gly Val Gly Val Pro Gly Xaa Gly Val Pro Gly Val
1               5                   10                  15

Gly Val Pro Gly Val Gly Val Pro Gly Val Gly Val Pro Gly Xaa Gly
            20                  25                  30

Val Pro Gly Val Gly Val Pro Gly Val Gly Val Pro Gly Val Gly Val
        35                  40                  45

Pro Gly Xaa Gly Val Pro Gly Val Gly Val Pro Gly Val Gly Val Pro
    50                  55                  60

Gly Val Gly Val Pro Gly Xaa Gly Val Pro Gly Val Gly Val Pro Gly
65                  70                  75                  80

Val Gly Val Pro Gly Val Gly Val Pro Gly Xaa Gly Val Pro Gly Val
                85                  90                  95

Gly Val Pro Gly Val Gly Val Pro Gly Val Gly Val Pro Gly Xaa Gly
            100                 105                 110

Val Pro Gly Val Gly Val Pro Gly Val Gly Val Pro Gly Val Gly Val
        115                 120                 125

Pro Gly Xaa Gly Val Pro Gly Val Gly Val Pro Gly Val Gly Val Pro
    130                 135                 140

Gly Val Gly Val Pro Gly Xaa Gly Val Pro Gly Val Gly Val Pro Gly
145                 150                 155                 160

Val Gly Val Pro Gly Val Gly Val Pro Gly Xaa Gly Val Pro Gly Val
                165                 170                 175

Gly Val Pro Gly Val Gly Val Pro Gly Val Gly Val Pro Gly Xaa Gly
            180                 185                 190

Val Pro Gly Val Gly Val Pro Gly Val Gly
        195                 200

<210>  7
<211>  302
<212>  PRT
<213>  Artificial sequence

<220>
<223>  Elastin-like polypeptide

<220>
<221>  MISC_FEATURE
<222>  (11)..(291)
<223>  Xaa denotes a sulfur-containing amino acid residue having a
       side-chain of formula -(CH2)z-S-R′ wherein z is 1 or 2 and R′ is
       a C1-C22 hydrocarbon radical

<400>  7

Met Trp Val Pro Gly Val Gly Val Pro Gly Xaa Gly Val Pro Gly Val
1               5                   10                  15

Gly Val Pro Gly Val Gly Val Pro Gly Val Gly Val Pro Gly Xaa Gly
            20                  25                  30

Val Pro Gly Val Gly Val Pro Gly Val Gly Val Pro Gly Val Gly Val
        35                  40                  45

Pro Gly Xaa Gly Val Pro Gly Val Gly Val Pro Gly Val Gly Val Pro
    50                  55                  60

Gly Val Gly Val Pro Gly Xaa Gly Val Pro Gly Val Gly Val Pro Gly
65                  70                  75                  80

Val Gly Val Pro Gly Val Gly Val Pro Gly Xaa Gly Val Pro Gly Val
                85                  90                  95

Gly Val Pro Gly Val Gly Val Pro Gly Val Gly Val Pro Gly Xaa Gly
            100                 105                 110

Val Pro Gly Val Gly Val Pro Gly Val Gly Val Pro Gly Val Gly Val
        115                 120                 125

Pro Gly Xaa Gly Val Pro Gly Val Gly Val Pro Gly Val Gly Val Pro
    130                 135                 140

Gly Val Gly Val Pro Gly Xaa Gly Val Pro Gly Val Gly Val Pro Gly
145                 150                 155                 160

Val Gly Val Pro Gly Val Gly Val Pro Gly Xaa Gly Val Pro Gly Val
                165                 170                 175

Gly Val Pro Gly Val Gly Val Pro Gly Val Gly Val Pro Gly Xaa Gly
            180                 185                 190

Val Pro Gly Val Gly Val Pro Gly Val Gly Val Pro Gly Val Gly Val
        195                 200                 205

Pro Gly Xaa Gly Val Pro Gly Val Gly Val Pro Gly Val Gly Val Pro
    210                 215                 220

Gly Val Gly Val Pro Gly Xaa Gly Val Pro Gly Val Gly Val Pro Gly
225                 230                 235                 240

Val Gly Val Pro Gly Val Gly Val Pro Gly Xaa Gly Val Pro Gly Val
                245                 250                 255

Gly Val Pro Gly Val Gly Val Pro Gly Val Gly Val Pro Gly Xaa Gly
            260                 265                 270

Val Pro Gly Val Gly Val Pro Gly Val Gly Val Pro Gly Val Gly Val
        275                 280                 285

Pro Gly Xaa Gly Val Pro Gly Val Gly Val Pro Gly Val Gly
    290                 295                 300

<210>  8
<211>  402
<212>  PRT
<213>  Artificial sequence

<220>
<223>  Elastin-like polypeptide

<220>
<221>  MISC_FEATURE
<222>  (11)..(391)
<223>  Xaa denotes a sulfur-containing amino acid residue having a
       side-chain of formula -(CH2)z-S-R′ wherein z is 1 or 2 and R′ is
       a C1-C22 hydrocarbon radical

<400>  8

Met Trp Val Pro Gly Val Gly Val Pro Gly Xaa Gly Val Pro Gly Val
1               5                   10                  15

Gly Val Pro Gly Val Gly Val Pro Gly Val Gly Val Pro Gly Xaa Gly
            20                  25                  30

Val Pro Gly Val Gly Val Pro Gly Val Gly Val Pro Gly Val Gly Val
        35                  40                  45

Pro Gly Xaa Gly Val Pro Gly Val Gly Val Pro Gly Val Gly Val Pro
    50                  55                  60

Gly Val Gly Val Pro Gly Xaa Gly Val Pro Gly Val Gly Val Pro Gly
65                  70                  75                  80

Val Gly Val Pro Gly Val Gly Val Pro Gly Xaa Gly Val Pro Gly Val
                85                  90                  95

Gly Val Pro Gly Val Gly Val Pro Gly Val Gly Val Pro Gly Xaa Gly
            100                 105                 110

Val Pro Gly Val Gly Val Pro Gly Val Gly Val Pro Gly Val Gly Val
        115                 120                 125

Pro Gly Xaa Gly Val Pro Gly Val Gly Val Pro Gly Val Gly Val Pro
    130                 135                 140

Gly Val Gly Val Pro Gly Xaa Gly Val Pro Gly Val Gly Val Pro Gly
145                 150                 155                 160

Val Gly Val Pro Gly Val Gly Val Pro Gly Xaa Gly Val Pro Gly Val
                165                 170                 175

Gly Val Pro Gly Val Gly Val Pro Gly Val Gly Val Pro Gly Xaa Gly
            180                 185                 190

Val Pro Gly Val Gly Val Pro Gly Val Gly Val Pro Gly Val Gly Val
        195                 200                 205

Pro Gly Xaa Gly Val Pro Gly Val Gly Val Pro Gly Val Gly Val Pro
    210                 215                 220

Gly Val Gly Val Pro Gly Xaa Gly Val Pro Gly Val Gly Val Pro Gly
225                 230                 235                 240

Val Gly Val Pro Gly Val Gly Val Pro Gly Xaa Gly Val Pro Gly Val
                245                 250                 255

Gly Val Pro Gly Val Gly Val Pro Gly Val Gly Val Pro Gly Xaa Gly
            260                 265                 270

Val Pro Gly Val Gly Val Pro Gly Val Gly Val Pro Gly Val Gly Val
        275                 280                 285

Pro Gly Xaa Gly Val Pro Gly Val Gly Val Pro Gly Val Gly Val Pro
    290                 295                 300

Gly Val Gly Val Pro Gly Xaa Gly Val Pro Gly Val Gly Val Pro Gly
305                 310                 315                 320

Val Gly Val Pro Gly Val Gly Val Pro Gly Xaa Gly Val Pro Gly Val
                325                 330                 335

Gly Val Pro Gly Val Gly Val Pro Gly Val Gly Val Pro Gly Xaa Gly
            340                 345                 350

Val Pro Gly Val Gly Val Pro Gly Val Gly Val Pro Gly Val Gly Val
        355                 360                 365

Pro Gly Xaa Gly Val Pro Gly Val Gly Val Pro Gly Val Gly Val Pro
    370                 375                 380

Gly Val Gly Val Pro Gly Xaa Gly Val Pro Gly Val Gly Val Pro Gly
385                 390                 395                 400

Val Gly

<210>  9
<211>  115
<212>  PRT
<213>  Artificial sequence

<220>
<223>  Elastin-like polypeptide

<400>  9

Met Gly Thr Glu Leu Ala Ala Ala Ser Glu Phe Thr His Met Trp Val
1               5                   10                  15

Pro Gly Met Gly Val Pro Gly Met Gly Val Pro Gly Met Gly Val Pro
            20                  25                  30

Gly Met Gly Val Pro Gly Met Gly Val Pro Gly Met Gly Val Pro Gly
        35                  40                  45

Met Gly Val Pro Gly Met Gly Val Pro Gly Met Gly Val Pro Gly Met
    50                  55                  60

Gly Val Pro Gly Met Gly Val Pro Gly Met Gly Val Pro Gly Met Gly
65                  70                  75                  80

Val Pro Gly Met Gly Val Pro Gly Met Gly Val Pro Gly Met Gly Val
                85                  90                  95

Pro Gly Met Gly Val Pro Gly Met Gly Val Pro Gly Met Gly Val Pro
            100                 105                 110

Gly Met Gly
        115

<210>  10
<211>  102
<212>  PRT
<213>  Artificial sequence

<220>
<223>  Elastin-like polypeptide

<400>  10

Met Trp Val Pro Gly Val Gly Val Pro Gly Met Gly Val Pro Gly Val
1               5                   10                  15

Gly Val Pro Gly Val Gly Val Pro Gly Val Gly Val Pro Gly Met Gly
            20                  25                  30

Val Pro Gly Val Gly Val Pro Gly Val Gly Val Pro Gly Val Gly Val
        35                  40                  45

Pro Gly Met Gly Val Pro Gly Val Gly Val Pro Gly Val Gly Val Pro
    50                  55                  60

Gly Val Gly Val Pro Gly Met Gly Val Pro Gly Val Gly Val Pro Gly
65                  70                  75                  80

Val Gly Val Pro Gly Val Gly Val Pro Gly Met Gly Val Pro Gly Val
                85                  90                  95

Gly Val Pro Gly Val Gly
            100

<210>  11
<211>  202
<212>  PRT
<213>  Artificial sequence

<220>
<223>  Elastin-like polypeptide

<400>  11

Met Trp Val Pro Gly Val Gly Val Pro Gly Met Gly Val Pro Gly Val
1               5                   10                  15

Gly Val Pro Gly Val Gly Val Pro Gly Val Gly Val Pro Gly Met Gly
                20                  25                  30

Val Pro Gly Val Gly Val Pro Gly Val Gly Val Pro Gly Val Gly Val
        35                  40                  45

Pro Gly Met Gly Val Pro Gly Val Gly Val Pro Gly Val Gly Val Pro
    50                  55                  60

Gly Val Gly Val Pro Gly Met Gly Val Pro Gly Val Gly Val Pro Gly
65                  70                  75                  80

Val Gly Val Pro Gly Val Gly Val Pro Gly Met Gly Val Pro Gly Val
                85                  90                  95

Gly Val Pro Gly Val Gly Val Pro Gly Val Gly Val Pro Gly Met Gly
            100                 105                 110

Val Pro Gly Val Gly Val Pro Gly Val Gly Val Pro Gly Val Gly Val
        115                 120                 125

Pro Gly Met Gly Val Pro Gly Val Gly Val Pro Gly Val Gly Val Pro
    130                 135                 140

Gly Val Gly Val Pro Gly Met Gly Val Pro Gly Val Gly Val Pro Gly
145                 150                 155                 160

Val Gly Val Pro Gly Val Gly Val Pro Gly Met Gly Val Pro Gly Val
                165                 170                 175

Gly Val Pro Gly Val Gly Val Pro Gly Val Gly Val Pro Gly Met Gly
            180                 185                 190

Val Pro Gly Val Gly Val Pro Gly Val Gly
        195                 200

<210>  12
<211>  302
<212>  PRT
<213>  Artificial sequence

<220>
<223>  Elastin-like polypeptide

<400>  12

Met Trp Val Pro Gly Val Gly Val Pro Gly Met Gly Val Pro Gly Val
1               5                   10                  15

Gly Val Pro Gly Val Gly Val Pro Gly Val Gly Val Pro Gly Met Gly
            20                  25                  30

Val Pro Gly Val Gly Val Pro Gly Val Gly Val Pro Gly Val Gly Val
        35                  40                  45

Pro Gly Met Gly Val Pro Gly Val Gly Val Pro Gly Val Gly Val Pro
    50                  55                  60

Gly Val Gly Val Pro Gly Met Gly Val Pro Gly Val Gly Val Pro Gly
65                  70                  75                  80

Val Gly Val Pro Gly Val Gly Val Pro Gly Met Gly Val Pro Gly Val
                85                  90                  95

Gly Val Pro Gly Val Gly Val Pro Gly Val Gly Val Pro Gly Met Gly
            100                 105                 110

Val Pro Gly Val Gly Val Pro Gly Val Gly Val Pro Gly Val Gly Val
        115                 120                 125

Pro Gly Met Gly Val Pro Gly Val Gly Val Pro Gly Val Gly Val Pro
    130                 135                 140

Gly Val Gly Val Pro Gly Met Gly Val Pro Gly Val Gly Val Pro Gly
145                 150                 155                 160

Val Gly Val Pro Gly Val Gly Val Pro Gly Met Gly Val Pro Gly Val
                165                 170                 175

Gly Val Pro Gly Val Gly Val Pro Gly Val Gly Val Pro Gly Met Gly
            180                 185                 190

Val Pro Gly Val Gly Val Pro Gly Val Gly Val Pro Gly Val Gly Val
        195                 200                 205

Pro Gly Met Gly Val Pro Gly Val Gly Val Pro Gly Val Gly Val Pro
    210                 215                 220

Gly Val Gly Val Pro Gly Met Gly Val Pro Gly Val Gly Val Pro Gly
225                 230                 235                 240

Val Gly Val Pro Gly Val Gly Val Pro Gly Met Gly Val Pro Gly Val
                245                 250                 255

Gly Val Pro Gly Val Gly Val Pro Gly Val Gly Val Pro Gly Met Gly
            260                 265                 270

Val Pro Gly Val Gly Val Pro Gly Val Gly Val Pro Gly Val Gly Val
        275                 280                 285

Pro Gly Met Gly Val Pro Gly Val Gly Val Pro Gly Val Gly
    290                 295                 300

<210>  13
<211>  402
<212>  PRT
<213>  Artificial sequence

<220>
<223>  Elastin-like polypeptide

<400>  13

Met Trp Val Pro Gly Val Gly Val Pro Gly Met Gly Val Pro Gly Val
1               5                   10                  15

Gly Val Pro Gly Val Gly Val Pro Gly Val Gly Val Pro Gly Met Gly
            20                  25                  30

Val Pro Gly Val Gly Val Pro Gly Val Gly Val Pro Gly Val Gly Val
        35                  40                  45

Pro Gly Met Gly Val Pro Gly Val Gly Val Pro Gly Val Gly Val Pro
    50                  55                  60

Gly Val Gly Val Pro Gly Met Gly Val Pro Gly Val Gly Val Pro Gly
65                  70                  75                  80

Val Gly Val Pro Gly Val Gly Val Pro Gly Met Gly Val Pro Gly Val
                85                  90                  95

Gly Val Pro Gly Val Gly Val Pro Gly Val Gly Val Pro Gly Met Gly
            100                 105                 110

Val Pro Gly Val Gly Val Pro Gly Val Gly Val Pro Gly Val Gly Val
        115                 120                 125

Pro Gly Met Gly Val Pro Gly Val Gly Val Pro Gly Val Gly Val Pro
    130                 135                 140

Gly Val Gly Val Pro Gly Met Gly Val Pro Gly Val Gly Val Pro Gly
145                 150                 155                 160

Val Gly Val Pro Gly Val Gly Val Pro Gly Met Gly Val Pro Gly Val
                165                 170                 175

Gly Val Pro Gly Val Gly Val Pro Gly Val Gly Val Pro Gly Met Gly
            180                 185                 190

Val Pro Gly Val Gly Val Pro Gly Val Gly Val Pro Gly Val Gly Val
        195                 200                 205

Pro Gly Met Gly Val Pro Gly Val Gly Val Pro Gly Val Gly Val Pro
    210                 215                 220

Gly Val Gly Val Pro Gly Met Gly Val Pro Gly Val Gly Val Pro Gly
225                 230                 235                 240

Val Gly Val Pro Gly Val Gly Val Pro Gly Met Gly Val Pro Gly Val
                245                 250                 255

Gly Val Pro Gly Val Gly Val Pro Gly Val Gly Val Pro Gly Met Gly
            260                 265                 270

Val Pro Gly Val Gly Val Pro Gly Val Gly Val Pro Gly Val Gly Val
        275                 280                 285

Pro Gly Met Gly Val Pro Gly Val Gly Val Pro Gly Val Gly Val Pro
    290                 295                 300

Gly Val Gly Val Pro Gly Met Gly Val Pro Gly Val Gly Val Pro Gly
305                 310                 315                 320

Val Gly Val Pro Gly Val Gly Val Pro Gly Met Gly Val Pro Gly Val
                325                 330                 335

Gly Val Pro Gly Val Gly Val Pro Gly Val Gly Val Pro Gly Met Gly
            340                 345                 350

Val Pro Gly Val Gly Val Pro Gly Val Gly Val Pro Gly Val Gly Val
        355                 360                 365

Pro Gly Met Gly Val Pro Gly Val Gly Val Pro Gly Val Gly Val Pro
    370                 375                 380

Gly Val Gly Val Pro Gly Met Gly Val Pro Gly Val Gly Val Pro Gly
385                 390                 395                 400

Val Gly